# EUROPEAN PATENT SPECIFICATION

(11) **EP 3 083 933 B1**
(45) Date of publication and mention of the grant of the patent: **21.01.2026**
(21) Application number: 14827357.6
(22) Date of filing: 19.12.2014
(51) Int. Cl.: C07K 16/00, C12N 5/00

(54) **USE OF PERFUSION SEED CULTURES TO IMPROVE BIOPHARMACEUTICAL FED-BATCH PRODUCTION CAPACITY AND PRODUCT QUALITY**
VERWENDUNG VON PERFUSIONSSAATKULTUREN ZUR VERBESSERUNG DER BIOPHARMAZEUTISCHEN FED-BATCH-PRODUKTIONSKAPAZITÄTEN UND DER PRODUKTQUALITÄT
UTILISATION DE CULTURES DE SEMENCES SOUS PERFUSION POUR AMÉLIORER LA CAPACITÉ DE PRODUCTION EN ALIMENTATION PROGRAMMÉE DE PRODUITS BIOPHARMACEUTIQUES ET LA QUALITÉ DES PRODUITS

(30) Priority: 20.12.2013 US 201361919629 P
(43) Date of publication of application: 26.10.2016
(73) Proprietor: Biogen MA Inc., Cambridge, MA 02142 (US)
(72) Inventor: YANG, William C., Rockville, MD 20852 (US); LU, Jiuyi, Ipswich, Massachusetts 01938 (US); HUANG, Yao-Ming, Cary, North Carolina 27519 (US); YUAN, Hang, Lexington, Massachusetts 02421 (US); KSHIRSAGAR, Rashmi, Ashland, Massachusetts 01721 (US); RYLL, Thomas, Lexington, Massachusetts 02421 (US)
(74) Representative: Mathys & Squire
(86) International application number: PCT/US2014/071701
(87) International publication number: WO 2015/095809

(56) References cited:
- WO-A1-2005/095578
- WO-A1-2009/129814
- WO-A2-02/50251
- WO-A2-2008/033517
- WO-A2-2013/006479
- US-A1- 2004 048 368
- R�DIGER HEIDEMANN ET AL: "A new seed-train expansion method for recombinant mammalian cell lines.", CYTOTECHNOLOGY, vol. 38, no. 1/3, 1 January 2002 (2002-01-01), Dordrecht, pages 99 - 108, XP055225875, ISSN: 0920-9069, DOI: 10.1023/A:1021114300958
- KARLHEINZ LANDAUER ED - RALF P�RTNER (ED): "Designing Media for Animal Cell Culture: CHO Cells, the Industrial Standard", 1 January 2014, ANIMAL CELL BIOTECHNOLOGY: METHODS AND PROTOCOLS IN: METHODS IN MOLECULAR BIOLOGY; ISSN; VOL. 1104; [METHODS IN MOLECULAR BIOLOGY; ISSN 1064-3745; VOL. 1104], HUMANA PRESS, US, PAGE(S) 89 - 103, ISBN: 978-1-62703-732-7, XP009185814
- ZIZHUO XING ET AL: "Optimizing amino acid composition of CHO cell culture media for a fusion protein production", PROCESS BIOCHEMISTRY, ELSEVIER LTD, GB, vol. 46, no. 7, 21 March 2011 (2011-03-21), pages 1423 - 1429, XP028223218, ISSN: 1359-5113, [retrieved on 20110329], DOI: 10.1016/J.PROCBIO.2011.03.014
- VEENA WARIKOO ET AL: "Integrated continuous production of recombinant therapeutic proteins", BIOTECHNOLOGY AND BIOENGINEERING, JOHN WILEY, HOBOKEN, USA, vol. 109, no. 12, 6 August 2012 (2012-08-06), pages 3018 - 3029, XP071129335, ISSN: 0006-3592, DOI: 10.1002/BIT.24584
- CHADERJIAN WENDY B ET AL: "Effect of copper sulfate on performance of a serum-free CHO cell culture process and the level of free thiol in the recombinant antibody expressed", BIOTECHNOLOGY PROGRESS, AMERICAN CHEMICAL SOCIETY, vol. 21, no. 2, 1 March 2005 (2005-03-01), pages 550 - 553, XP002500111, ISSN: 8756-7938, DOI: 10.1021/BP0497029
- PADAWER ISHAI ET AL: "Case Study: An accelerated 8-day monoclonal antibody production process based on high seeding densities", BIOTECHNOLOGY PROGRESS, vol. 29, no. 3, May 2013 (2013-05-01), pages 829 - 832, XP002735825
- POHLSCHEIDT MICHAEL ET AL: "Optimizing capacity utilization by large scale 3000 L perfusion in seed train bioreactors", BIOTECHNOLOGY PROGRESS, 1 January 2012 (2012-01-01), pages n/a - n/a, XP055049926, ISSN: 8756-7938, DOI: 10.1002/btpr.1672
- GARGI SETH ET AL: "Development of a new bioprocess scheme using frozen seed train intermediates to initiate CHO cell culture manufacturing campaigns", BIOTECHNOLOGY AND BIOENGINEERING, vol. 110, no. 5, 4 May 2013 (2013-05-04), pages 1376 - 1385, XP055168927, ISSN: 0006-3592, DOI: 10.1002/bit.24808
- MARIE-FRANÇOISE CLINCKE ET AL: "Very high density of Chinese hamster ovary cells in perfusion by alternating tangential flow or tangential flow filtration in WAVE bioreactor(TM)-part II: Applications for antibody production and cryopreservation", BIOTECHNOLOGY PROGRESS, vol. 29, no. 3, 21 May 2013 (2013-05-21), pages 768 - 777, XP055168922, ISSN: 8756-7938, DOI: 10.1002/btpr.1703
- YAO-MING HUANG ET AL: "Maximizing productivity of CHO cell-based fed-batch culture using chemically defined media conditions and typical manufacturing equipment", BIOTECHNOLOGY PROGRESS, vol. 26, no. 5, 22 September 2010 (2010-09-22), pages 1400 - 1410, XP055168194, ISSN: 8756-7938, DOI: 10.1002/btpr.436
- WILLIAM C. YANG ET AL: "Perfusion seed cultures improve biopharmaceutical fed-batch production capacity and product quality", BIOTECHNOLOGY PROGRESS, vol. 30, no. 3, 1 May 2014 (2014-05-01), pages 616 - 625, XP055168419, ISSN: 8756-7938, DOI: 10.1002/btpr.1884

## Description

### Field of the Invention

The present disclosure relates to methods of improving the efficiency of production of a bioproduct of interest in a mammalian cell culture. In particular, the methods increase the quantity of a bioproduct produced, and/or decrease bioproduct production time in a manufacturing-scale production bioreactor cell culture. The disclosed methods comprise: (a) culturing the cells in a N-1 seed train (growth stage) bioreactor cell culture to obtain high viable cell densities; and (b) seeding the production bioreactor culture at high viable cell seeding densities. The present invention relates to a method of producing a protein of interest in a CHO cell culture, as set out in the appended claims.

### BACKGROUND OF THE INVENTION

### Background Art

The production bioreactor is one of the bottlenecks in bioproduct manufacturing. Traditional batch or fed-batch production processes consist of an unproductive growth phase, when cell mass accumulates, followed by a more productive stationary phase, when the majority of the bioproduct is generated. The unproductive growth phase lengthens the duration and lowers the volumetric productivity of the production process, which in turn leads to inefficient production bioreactor utilization and reduces the output rate. Improvements in volumetric productivity and production bioreactor usage have been seen by shifting the growth phase from the production stage bioreactor into the N-1 seed train stage (growth stage) bioreactor. (Pohlscheidt et al., Biotechnology Progress, 29(1), 222-9 (2013); Padawer et al., Biotechnology Progress, 29(3), 829-832 (2013)).

It is, however, difficult to obtain high viable cell densities in N-1 seed train stage. Traditional batch or fed-batch N-1 seed train cultures cannot sustain high viable cell densities. Thus, there is a need in the art for improved methods for culturing cells in a bioreactor.

### SUMMARY OF THE INVENTION

The present invention provides a method of producing a protein of interest, comprising:
(a) culturing CHO cells comprising a gene that encodes the protein of interest in a N-1 culture vessel to achieve a cell density of at least 25 x 10⁶ viable cells/ml;
(b) inoculating a N culture vessel at a seeding density of at least 8.5 x 10⁶ viable cells/ml with cells obtained from step (a); and
(c) culturing the cells in the N culture vessel under conditions that allow production of the protein of interest,

wherein the culture in step (a) is supplemented with nutrients at a level determined based on the viable cell density,
wherein waste products are removed during step (a) and/or the culture in the N-1 culture vessel of step (a) is perfused, and
wherein lactate levels are maintained below 15 mM in the culture of step (a).

The present invention and embodiments thereof are set out in the appended claims.

### DETAILED DESCRIPTION OF THE INVENTION

The technical information set out below may in some respects go beyond the scope of the invention, which is defined exclusively by the appended claims. The additional technical information is provided to place the actual invention in a broader technical context and to illustrate possible related technical developments.

The present invention is related to methods for obtaining high viable cell density in the N-1 seed train stage by nutrient supplementation, and waste product removal or using a perfusion cell culture process in the N-1 seed train bioreactor. A high viable cell density in the N-1 seed train bioreactor allows for a higher viable cell seeding density in the production bioreactor. By the invention, the high-seed production bioreactor delivers the same or higher titer of the product in a shorter culture duration, leading to an increased N-1 seed train bioreactor occupancy per run and reduced production bioreactor occupancy per run. The shorter production duration and higher volumetric productivity can yield more production batches per run and lead to increased manufacturing capacity with few changes to the batch or fed-batch production bioreactor equipment.

The present invention is related to a method of producing a protein, comprising: (a) culturing CHO cells comprising a gene that encodes the protein of interest in a N-1 culture vessel to achieve a cell density of at least 25 x 10⁶ viable cells/ml; (b) inoculating a N culture vessel at a seeding density of at least 8.5 x 10⁶ viable cells/ml with cells obtained from step (a); and (c) culturing the cells in the N culture vessel under conditions that allow production of the protein of interest, wherein the culture in step (a) is supplemented with nutrients at a level determined based on the viable cell density, wherein waste products are removed during step (a) and/or the culture in the N-1 culture vessel of step (a) is perfused, and wherein lactate levels are maintained below 15 mM in the culture of step (a).

In some embodiments, during step (a), the viable cell density of the culture is periodically determined. In some embodiments, the nutrients comprise amino acids, zinc, iron, and copper. In some embodiments, zinc is added to the cell culture of step (a) in a cumulative amount of between about 1.8 µM to about 1 mM. In some embodiments, iron is added to the cell culture of step (a) in a cumulative amount of between about 0.1 µM to about 10 mM. In some embodiments, copper is added to the cell culture of step (a) in a cumulative amount of between 0.008 µM to about 1 mM. In some embodiments, amino acids are added to the cell culture of step (a) in a cumulative amount of about 50 mM to about 2 M.

In some embodiments, waste products are removed during step (a) of the present invention In certain aspects, the waste removal is performed by filtration, centrifugation, an inclined cell settler, alternating tangential flow, tangential flow, or combinations thereof.

In certain embodiments, the waste products are removed during step (a) and amino acids are added in a cumulative amount of about 75 mM to about 500 mM. In certain embodiments, the waste products are removed during step (a) and amino acids are added in a cumulative amount of about 75 mM to about 800 mM.

In some embodiments, the lactate level in step (a) is maintained below 10 mM, 1 mM, or 0.1 mM.

The cells in step (a) of the method are cultured to high cell densities. In some embodiments, the cells in step (a) of the method are cultured to high viable cell densities. In some embodiments, a cell density of at least 30 x 10⁶ viable cells/ml is achieved during step (a). In other embodiments, cell densities of at least 35 x 10⁶ viable cells/ml, at least 40 x 10⁶ viable cells/ml, at least 45 x 10⁶ viable cells/ml, at least 50 x 10⁶ viable cells/ml, at least 55 x 10⁶ viable cells/ml, at least 60 x 10⁶ viable cells/ml, or at least 65 x 10⁶ cells/ml are achieved during step (a).

In some embodiments, the viable cell density of the culture in step (a) is determined about ten times per second. In another embodiment, the viable cell density of the culture in step (a) is determined about nine times per second. In another embodiment, the viable cell density of the culture in step (a) is determined about eight times per second. In another embodiment, the viable cell density of the culture in step (a) is determined about seven times per second. In another embodiment, the viable cell density of the culture in step (a) is determined about six times per second. In another embodiment, the viable cell density of the culture in step (a) is determined about five times per second. In another embodiment, the viable cell density of the culture in step (a) is determined about four times per second. In another embodiment, the viable cell density of the culture in step (a) is determined about three times per second. In another embodiment, the viable cell density of the culture in step (a) is determined about twice per second. In another embodiment, the viable cell density of the culture in step (a) is determined about once per second. In another embodiment, the viable cell density of the culture in step (a) is determined about once per minute. In another embodiment, the viable cell density of the culture in step (a) is determined about once per hour. In another embodiment, the viable cell density of the culture in step (a) is determined about four times per day. In another embodiment, the viable cell density of the culture in step (a) is determined about three times per day. In another embodiment, the viable cell density of the culture in step (a) is determined about twice per day. In another embodiment, the viable cell density of the culture in step (a) is determined about once per day. In other embodiments, the viable cell density of the culture in step (a) is determined continuously.

In some embodiments, the cell culture of step (a) is performed as a perfusion cell culture. In certain embodiments, the perfusion rate in the perfusion cell culture is about 0.10 nL/cell/day. In other embodiments, the perfusion rate in the perfusion cell culture is about 0.07 nL/cell/day. In other embodiments, the perfusion rate in the perfusion cell culture is about 0.05 nL/cell/day. In some embodiments, the perfusion in perfusion cell culture is alternating tangential flow. In other embodiments, the perfusion in the perfusion cell culture is tangential flow.

In some embodiments, the cell culture of step (a) is maintained for about 3 days to about 8 days.

In some embodiments, the N culture vessel in step (b) is inoculated at a seeding density of about 8.5 x 10⁶ viable cells/ml. In other embodiments, the N culture vessel in step (b) is inoculated at a seeding density of about 10 x 10⁶ viable cells/ml, about 15 x 10⁶ viable cells/ml, 20 x 10⁶ viable cells/ml, 25 x 10⁶ viable cells/ml, or 30 x 10⁶ viable cells/ml.

In some embodiments, the culture of step (c) is performed as a fed-batch culture. In other embodiments, the culture of step (c) is performed as a perfusion culture.

In some embodiments, the culture of step (c) is maintained for about 8 days to about 23 days.

In some embodiments, the methods further comprise the step of collecting the protein of interest produced by the cells in the N culture vessel.

In some embodiments, the protein product of interest is an antibody. In certain embodiments, the protein product of interest is selected from antibody, fusion protein, alpha-synuclein, BART, Lingo, aBDCA2, anti-CD40L, STX-100, Tweak, daclizumab, pegylated interferon, interferon, etanercept, infliximab, trastuzumab, adalimumab, bevacizumab, Tysabri, Avonex, Rituxan, ocrelizumab, obinutuzumab, or any other protein that binds to CD20.

In some embodiments, the volume of the N-1 culture vessel in step (a) is less about 50 liters to about 20,000 liters. In other embodiments, the volume of the N-1 culture vessel in step (a) is about 100 liters to about 4000 liters.

In some embodiments, the volume of the N culture vessel in step (b) is between about 200 liters to about 20,000 liters.

In some embodiments, the cell cultures of step (a) and step (c) are grown at different temperatures. In certain embodiments, the cell culture of step (c) is grown at a lower temperature than the culture of step (a). In some embodiments, the cell culture of step (c) is grown at about 30°C and the cell culture of step (a) is grown at about 36°C.

In some embodiments, the culture of step (a) is grown at a pH of about 6.8 to about 7.4. In other embodiments, the culture of step (a) is grown at a pH of about 6.8 to about 7.3.

In some embodiments, the present invention is directed to a method of efficiently producing an antibody.

The invention can also be directed to a bioproduct produced by the method of the present invention. In some embodiments, the bioproduct is an antibody or antibody-like polypeptide.

### BRIEF DESCRIPTION OF THE DRAWINGS/FIGURES

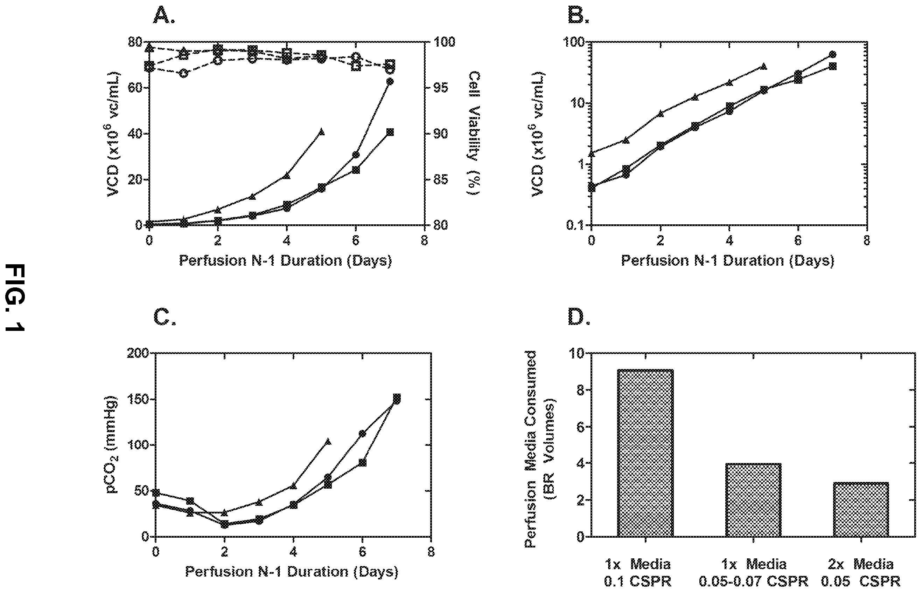

FIG. 1A-D: Optimization of Perfusion in N-1 Cultures for Cell Line A. Shortening the process duration, lowering the CSPR, and concentrating the perfusion media lead to better growth (A, B), lower pCO2 (C), and lower perfusion volume consumption (D). All data were N=1. Open markers in (A) denote cell viability. • = 1x Media, 0.1 CSPR ■ = 1x Media, 0.05-0.07 CSPR ▲ = 2x Media, 0.05 CSPR.

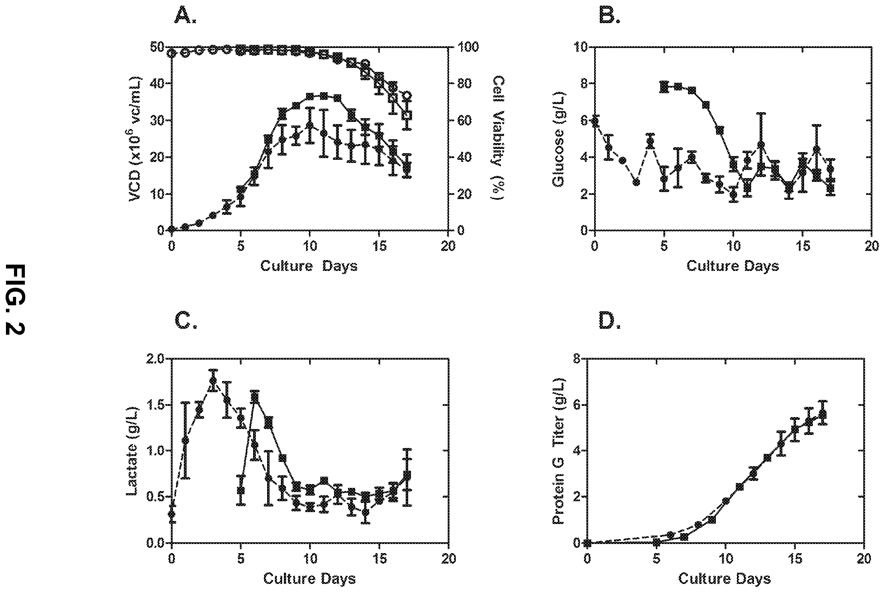

FIG. 2A-D: High-Seed Fed-batch Production Cultures for Cell Line A. High-seed growth (A), metabolism (B, C), and protein production (D) track that of the corresponding low-seed process. All data were at least N=2. Open markers in (A) denote cell viability. • = Low-seed process ■= High-seed process

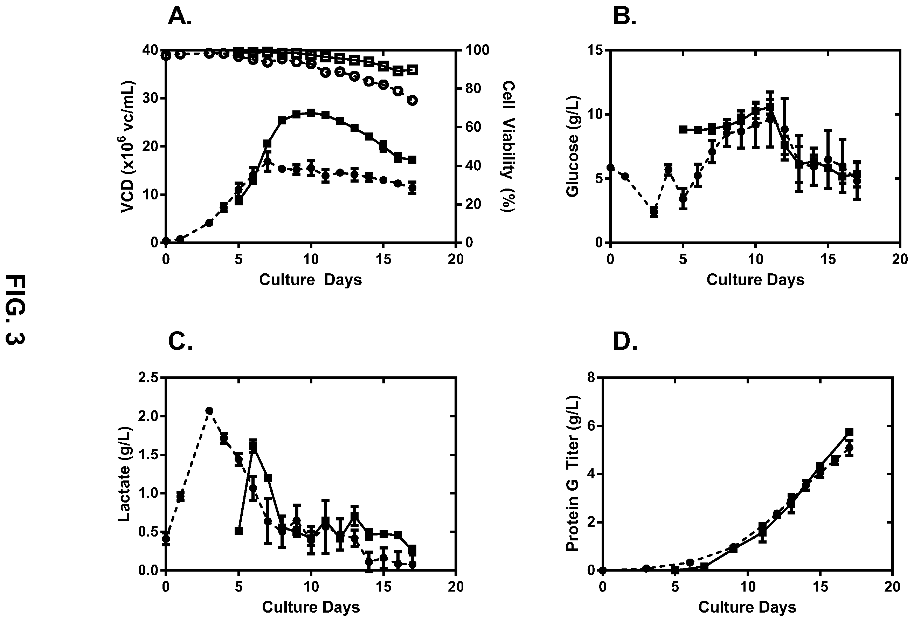

FIG. 3A-D: High-Seed Fed-batch Production Cultures for Cell Line B. High-seed growth (A), metabolism (B, C), and protein production (D) track that of the corresponding low-seed process. All data were at least N=2. Open markers in (A) denote cell viability. • = Low-seed process ■ = High-seed process

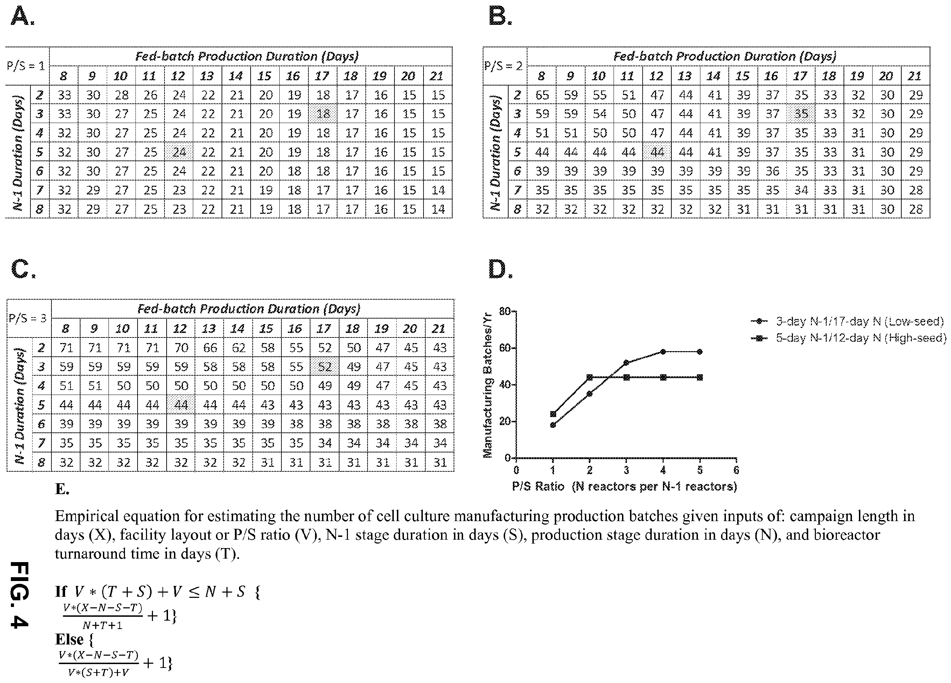

FIG. 4A-D: Effect of facility layout, seed train duration, and production bioreactor duration on manufacturing capacity. The annual capacity/output (number of batches per year) was calculated from an empirical equation shown in FIG. 4E. Highlighted cells correspond to the output for the low-seed (3-day N-1/17-day N) and high-seed (5-day N-1/12-day N) production regimes. Campaign length was assumed to be 365 days and bioreactor turnaround time was assumed to be 2 days. The annual capacity in terms of production batches is shown for P/S ratios of 1 (A), 2 (B), and 3 (C). (D) shows the annual capacity of the low-seed and high-seed process for cell lines A and B as a function of the P/S ratio. Increasing the number of production vessels per seed train results in diminishing returns as the bottleneck shifts to the seed train.

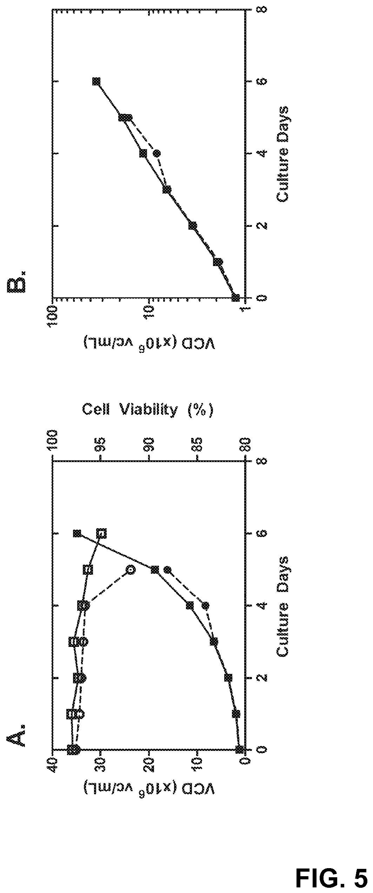

FIG. 5A-B: Perfusion in N-1 Cultures for Cell Line C. Open markers in (A) denote cell viability. • = 2x Media, 0.05-0.10 CSPR ■ = 1x Media, 0.10-12 CSPR

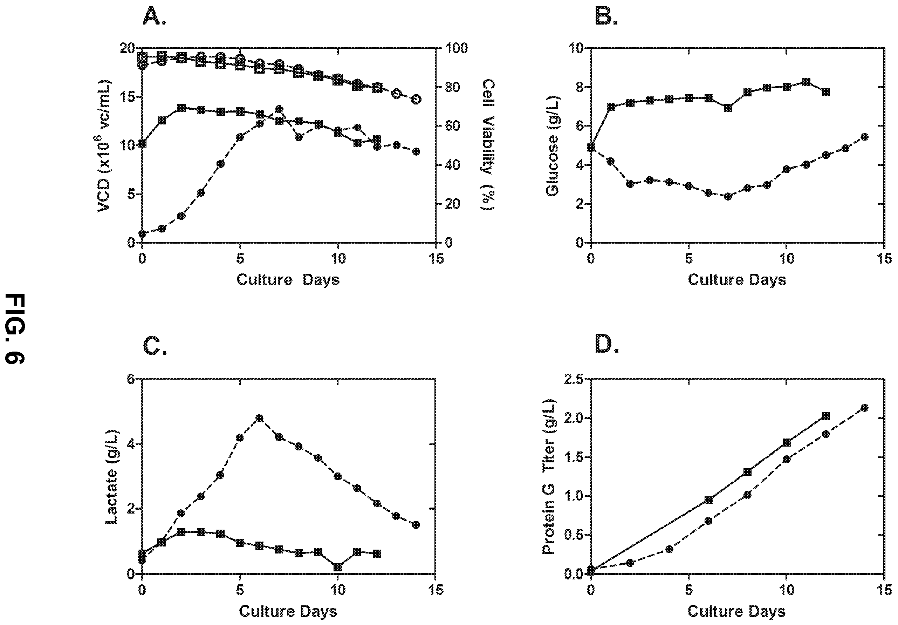

FIG. 6A-D: High-Seed Fed-batch Production Cultures for Cell Line C. High-seed results in a completely different growth (A), metabolism (B, C), and protein production (D) profile compared to the corresponding low-seed process due to the differences in starting temperature. All data were N=1. Open markers in (A) denote cell viability. • = Low-seed process ■ = High-seed process.

### FURTHER DETAILED DESCRIPTION OF THE INVENTION

The present disclosure is related to methods of improving volumetric productivity and product quality of bioproduct cell culture process by shifting the growth phase of the process from the production stage to the N-1 seed train stage, by providing nutrient supplementation and/or waste product removal, or optionally using a perfusion process in the N-1 seed train stage culture. The method of the present invention comprises nutrient supplementation in the N-1 seed train stage culture, and the waste products are removed in the N-1 culture vessel and/or the culture in the N-1 culture vessel is perfused.

The method of the invention attains high viable cell densities in the N-1 seed train stage, which allows for a high-seed production stage. The high-seed production stage can deliver the same or higher titer of the bioproduct in a shorter culture duration. The improved culture method of the present invention provide increased occupancy at the seed train stage and reduced occupancy at the production stage per run.

The disclosure is related to a method of producing a bioproduct, comprising: (a) culturing mammalian cells comprising a gene that encodes the bioproduct of interest in a N-1 culture vessel to achieve a cell density of at least 25 x 10⁶ viable cells/ml; (b) inoculating a N culture vessel at a seeding density of at least 8.5 x 10⁶ viable cells/ml with cells obtained from step (a); and (c) culturing the cells in the N culture vessel under conditions that allow production of the protein of interest. The present invention is related to a method of producing a protein of interest comprising culturing CHO cells.

The present invention also relates to a bioproduct produced by the method of the present invention.

### I. DEFINITIONS

It is to be noted, unless otherwise clear from the context, that the term "a" or "an" entity refers to one or more of that entity; for example, "an amino acid," is understood to represent one or more amino acids. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following embodiments: A, B, and C; A, B, or C; A or C; A or B; B or C; A and C; A and B; B and C; A (alone); B (alone); and C (alone).

It is understood that wherever embodiments are described with the language "comprising," otherwise analogous embodiments described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various embodiments of the disclosure, which can be had by reference to the specification as a whole. Accordingly, the terms defined immediately below are more fully defined by reference to the specification in its entirety.

The terms "media", "medium", "cell culture medium", "culture medium", "tissue culture medium", "tissue culture media", and "growth medium" as used herein refer to a solution containing nutrients which nourish growing cultured eukaryotic cells. Typically, these solutions provide essential and non-essential amino acids, vitamins, energy sources, lipids, and trace elements required by the cell for minimal growth and/or survival. The solution can also contain components that enhance growth and/or survival above the minimal rate, including hormones and growth factors. The solution is formulated to a pH and salt concentration optimal for cell survival and proliferation. The medium can also be a "defined medium" or "chemically defined medium"--a serum-free medium that contains no proteins, hydrolysates or components of unknown composition. Defined media are free of animal-derived components and all components have a known chemical structure. One of skill in the art understands a defined medium can comprise recombinant glycoproteins or proteins, for example, but not limited to, hormones, cytokines, interleukins and other signaling molecules.

The term "basal media formulation" or "basal media" as used herein refers to any cell culture media used to culture cells that has not been modified either by supplementation, or by selective removal of a certain component.

The terms "culture", "cell culture" and "eukaryotic cell culture" as used herein refer to a eukaryotic cell population, either surface-attached or in suspension that is maintained or grown in a medium (see definition of "medium" below) under conditions suitable to survival and/or growth of the cell population. As will be clear to those of ordinary skill in the art, these terms as used herein can refer to the combination comprising the mammalian cell population and the medium in which the population is suspended.

The term "batch culture" as used herein refers to a method of culturing cells in which all the components that will ultimately be used in culturing the cells, including the medium (see definition of "medium" below) as well as the cells themselves, are provided at the beginning of the culturing process. A batch culture is typically stopped at some point and the cells and/or components in the medium are harvested and optionally purified.

The term "fed-batch culture" as used herein refers to a method of culturing cells in which additional components are provided to the culture at some time subsequent to the beginning of the culture process. A fed-batch culture can be started using a basal medium. The culture medium with which additional components are provided to the culture at some time subsequent to the beginning of the culture process is a feed medium. The provided components typically comprise nutritional supplements for the cells which have been depleted during the culturing process. A fed-batch culture is typically stopped at some point and the cells and/or components in the medium are harvested and optionally purified.

The term "perfusion culture" as used herein refers to a method of culturing cells in which additional components are provided continuously or semi-continuously to the culture subsequent to the beginning of the culture process. The provided components typically comprise nutritional supplements for the cells which have been depleted during the culturing process. A portion of the cells and/or components in the medium are typically harvested on a continuous or semi-continuous basis and are optionally purified.

"Growth phase" of the cell culture refers to the period of exponential cell growth (the log phase) where cells are generally rapidly dividing. During this phase, cells are cultured for a period of time, usually between 1-4 days, and under such conditions that cell growth is maximized. The determination of the growth cycle for the host cell can be determined for the particular host cell envisioned without undue experimentation. "Period of time and under such conditions that cell growth is maximized" and the like, refer to those culture conditions that, for a particular cell line, are determined to be optimal for cell growth and division. During the growth phase, cells are cultured in nutrient medium containing the necessary additives generally at about 25°-40°C, in a humidified, controlled atmosphere, such that optimal growth is achieved for the particular cell line. Cells are maintained in the growth phase for a period of about between one and seven days, e.g., between two to six days, e.g., six days. The length of the growth phase for the particular cells can be determined without undue experimentation. For example, the length of the growth phase will be the period of time sufficient to allow the particular cells to reproduce to a viable cell density within a range of about 20% -80% of the maximal possible viable cell density if the culture was maintained under the growth conditions.

"Production phase" or "protein production phase" of the cell culture refers to the period of time during which cell growth has plateaued. During the production phase, logarithmic cell growth has ended and bioproduct production is primary. During this period of time, the medium is generally supplemented to support continued bioproduct production and to achieve the desired bioproduct.

The term "cell viability" as used herein refers to the ability of cells in culture to survive under a given set of culture conditions or experimental variations. The term as used herein also refers to that portion of cells which are alive at a particular time in relation to the total number of cells, living and dead, in the culture at that time.

The term "cell density" as used herein refers to that number of cells present in a given volume of medium.

The term "bioreactor" or "culture vessel" as used herein refers to any vessel used for the growth of a mammalian cell culture. The bioreactor can be of any size so long as it is useful for the culturing of mammalian cells.

As used herein, the term "bioreactor run" can include one or more of the lag phase, log phase, or plateau phase growth periods during a cell culture cycle.

The term "first culture vessel," "N-1 culture vessel," "N-1 seed-train culture vessel," "N-1 vessel," "first bioreactor," "N-1 bioreactor," "N-1 seed-train bioreactor" as used herein refers to a culture vessel that is immediately before the N culture vessel (production culture vessel) and is used to grow the cell culture to a high viable cell density for subsequent inoculation into N (production) culture vessel. The cell culture to be grown in the N-1 culture vessel may be obtained after culturing the cells in several vessels prior to the N-1 culture vessel, such as N-4, N-3, and N-2 vessels.

The terms "N culture vessel," "second culture vessel," "production culture vessel," "N vessel," "N bioreactor," "second bioreactor," "production bioreactor" as used herein refers to the bioreactor after the N-1 bioreactor and is used in the production of the bioproduct of interest.

The term "traditional manufacturing process," as used herein, refers to either the traditional processes of (a) adding nutrient media in bolus feeds to the bioreactor at designated time points, or (b) adding glucose (or another single nutrient) to the bioreactor as the glucose (or other single nutrient) is consumed. The traditional manufacturing processes lead to lower bioproduct yields and/or less efficient bioproduct production.

The term "seeding" as used herein refers to the process of providing a cell culture to a bioreactor or another vessel. In one embodiment, the cells have been propagated previously in another bioreactor or vessel. In another embodiment, the cells have been frozen and thawed immediately prior to providing them to the bioreactor or vessel. The term refers to any number of cells, including a single cell.

The term "titer" as used herein refers to the total amount of recombinantly expressed glycoprotein or protein produced by a cell culture divided by a given amount of medium volume. Titer is typically expressed in units of milligrams of glycoprotein or protein per milliliter of medium or in units of grams of glycoprotein or protein per liter of medium.

As used herein, the terms "additive" or "supplement" or "nutrient" refer to any compound, molecule, or substance used by an organism, such as a cell, in culture, to live, grow, or otherwise add biomass. Examples of nutrients include carbohydrates(e.g., simple sugars such as glucose, galactose, maltose or fructose, or more complex sugars), amino acids, vitamins (e.g., B group vitamins (e.g., B12), vitamin A vitamin E, riboflavin, thiamine and biotin), zinc, iron, or copper. In certain embodiments, nutrient is supplemented to a basal medium to achieve the goals described in this disclosure. An "additive" or "supplement" or "nutrient" can include a single substance, e.g., copper, zinc, amino acid, or can include multiple substances, e.g., zinc and amino acid. The terms "additive" or "supplement" or "nutrient" refer to all of the components added, even though they need not be added at the same time, and they need not be added in the same way. For example, one or more components of an "additive" or "supplement" or "supplement" can be added as a single bolus or two or more boli from a stock solution, while other components of the same "additive" or "supplement" or "nutrient" can be added as part of a feed medium. In addition, any one or more components of an "additive" or "supplement" or "nutrient" can be present in the basal medium from the beginning of the cell culture.

The term "amino acid" as used herein refers any of the twenty standard amino acids, *i.e.*, glycine, alanine, valine, leucine, isoleucine, methionine, proline, phenylalanine, tryptophan, serine, threonine, asparagine, glutamine, tyrosine, cysteine, lysine, arginine, histidine, aspartic acid and glutamic acid, single stereoisomers thereof, and racemic mixtures thereof. The term "amino acid" can also refer to the known non-standard amino acids, e.g., 4-hydroxyproline, ε-*N*,*N*,*N*-trimethyllysine, 3-methylhistidine, 5-hydroxylysine, *O*-phosphoserine, γ-carboxyglutamate, ε-*N*-acetyllysine, ω-*N-*methylarginine, *N*-acetylserine, *N*,*N,N*-trimethylalanine, *N*-formylmethionine, γ-aminobutyric acid, histamine, dopamine, thyroxine, citrulline, ornithine, β-cyanoalanine, homocysteine, azaserine, and S-adenosylmethionine. In some embodiments, the amino acid is glutamate, glutamine, lysine, tyrosine or valine. In some embodiments, the amino acid is glutamate or glutamine.

The term "nutrient media," "feed media," "feed," "total feed," and "total nutrient media" as used herein can be used interchangeably, and include a "complete" media used to grow, propagate, and add biomass to a cell line. Nutrient media is distinguished from a substance or simple media which by itself is not sufficient to grow and propagate a cell line. Thus, for example, glucose or simple sugars by themselves are not nutrient media, since in the absence of other required nutrients, they would not be sufficient to grow and propagate a cell line. One of skill in the art can appreciate that cells may continue to grow, live and propagate in the presence of incomplete media, but become instable and/or greatly reduce their growth rate. Thus, in some embodiments, the term "nutrient media" includes a media sufficient to grow, propagate, and add biomass to a cell line without a loss in stability, growth rate, or a reduction of any other indicators of cellular health for a period of at least 2 days, 3 days, 4 days, 5 days, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, or 18 weeks. In some embodiments, the term "nutrient media" includes a media which may lack one or more essential nutrients, but which can continue to grow, propagate, and add biomass to a cell line without a loss in stability, growth rate, or a reduction of any other indicators of cellular health for a period of at least 2 days, 3 days, 4 days, 5 days, 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, or 18 weeks.

In some embodiments, the nutrient media is a cell culture media. Optimal cell culture media compositions vary according to the type of cell culture being propagated. In some embodiments, the nutrient media is a commercially available media. In some embodiments, the nutrient media contains e.g., inorganic salts, carbohydrates (e.g., sugars such as glucose, galactose, maltose or fructose), amino acids, vitamins (e.g., B group vitamins (e.g., B12), vitamin A vitamin E, riboflavin, thiamine and biotin), fatty acids and lipids (e.g., cholesterol and steroids), proteins and peptides (e.g., albumin, transferrin, fibronectin and fetuin), serum (e.g., compositions comprising albumins, growth factors and growth inhibitors, such as, fetal bovine serum, newborn calf serum and horse serum), trace elements (e.g., zinc, copper, selenium and tricarboxylic acid intermediates), hydrolysates (hydrolyzed proteins derived from plant or animal sources), and combinations thereof. Examples of nutrient medias include, but are not limited to, basal media (e.g., MEM, DMEM, GMEM), complex media (RPMI 1640, Iscoves DMEM, Leibovitz L-15, Leibovitz L-15, TC 100), serum free media (e.g., CHO, Ham F10 and derivatives, Ham F12, DMEM/F12). Common buffers found in nutrient media include PBS, Hanks BSS, Earles salts, DPBS, HBSS, and EBSS. Media for culturing mammalian cells are well known in the art and are available from, e.g., Sigma-Aldrich Corporation (St. Louis, MO), HyClone (Logan, UT), Invitrogen Corporation (Carlsbad, CA), Cambrex Corporation (E. Rutherford, NJ), Irvine Scientific (Santa Ana, CA), and others. Other components found in nutrient media can include ascorbate, citrate, cysteine/cystine, glutamine, folic acid, glutathione, linoleic acid, linolenic acid, lipoic acid, oleic acid, palmitic acid, pyridoxal/pyridoxine, riboflavin, selenium, thiamine, and transferrin. One of skill in the art will recognize that there are modifications to nutrient media which would fall within the scope of this invention.

The term "bioproduct" as used herein refers to a molecule that is produced an can be isolated from cellular culture, e.g., eukaryotic (e.g., mammalian) cell culture. In some embodiments, the term bioproduct refers to molecules with a molecular mass exceeding 50 kDa, 75 kDa, 100 kDa, 125 kDa, or 150 kDa. In some embodiments, the use of the term refers to polymers, e.g., biopolymers such as nucleic acids (such as DNA, RNA), polypeptides (such as proteins), carbohydrates, and lipids. In some embodiments, the term "bioproduct" refers to a protein. In some embodiments, the term "bioproduct" refers to a recombinant protein or a fusion protein. In some embodiments, the bioproduct is a soluble protein. In some embodiments, the bioproduct is an antibody, antibody fragment or modified antibody (e.g., a multivalent antibody, a domain-deleted antibody, a multimeric antibody, a hinge-modified antibody, a stabilized antibody, a multispecific antibody, a linear antibody, an scFv, a linked scFv antibody, a multivalent linear antibody, a multivalent antibody without Fc, a Fab, a multivalent Fab, etc.), a monoclonal antibody or a polyclonal antibody.

The terms "polypeptide" or "protein" as used herein refers a sequential chain of amino acids linked together via peptide bonds. The term is used to refer to an amino acid chain of any length, but one of ordinary skill in the art will understand that the term is not limited to lengthy chains and can refer to a minimal chain comprising two amino acids linked together via a peptide bond. If a single polypeptide is the discrete functioning unit and does require permanent physical association with other polypeptides in order to form the discrete functioning unit, the terms "polypeptide" and "protein" as used herein are used interchangeably. If discrete functional unit is comprised of more than one polypeptide that physically associate with one another, the term "protein" as used herein refers to the multiple polypeptides that are physically coupled and function together as the discrete unit. The term "protein" as used herein is intended to encompass a singular "protein" as well as plural "proteins." Thus, as used herein, terms including, but not limited to "peptide," "polypeptide," "amino acid chain," or any other term used to refer to a chain or chains of amino acids, are included in the definition of a "protein," and the term "protein" may be used instead of, or interchangeably with, any of these terms. The term further includes proteins which have undergone post-translational modifications, for example, glycosylation, acetylation, phosphorylation, amidation, derivatization by known protecting/blocking groups, proteolytic cleavage, or modification by non-naturally occurring amino acids. Proteins also include polypeptides which form multimers, e.g., dimers, trimers, etc. The term protein also includes fusions proteins, e.g., a protein that is produced via a gene fusion process in which a protein (or fragment of a protein) is attached to an antibody (or fragment of antibody). Examples of fusion proteins of the present invention include disulfide-linked bifunctional proteins comprised of linked Fc regions from human IgG1 and human IgE; and lymphotoxin beta receptor immunoglobulin G1.

The term "antibody" is used to mean an immunoglobulin molecule that recognizes and specifically binds to a target, such as a protein, polypeptide, peptide, carbohydrate, polynucleotide, lipid, or combinations of the foregoing etc., through at least one antigen recognition site within the variable region of the immunoglobulin molecule. As used herein, the term encompasses intact polyclonal antibodies, intact monoclonal antibodies, antibody fragments (such as Fab, Fab', F(ab')2, and Fv fragments), single chain Fv (scFv) mutants, multispecific antibodies such as bispecific antibodies generated from at least two intact antibodies, monovalent or monospecific antibodies, chimeric antibodies, humanized antibodies, human antibodies, fusion proteins comprising an antigen determination portion of an antibody, and any other modified immunoglobulin molecule comprising an antigen recognition site so long as the antibodies exhibit the desired biological activity. An antibody can be any of the five major classes of immunoglobulins: IgA, IgD, IgE, IgG, and IgM, or subclasses (isotypes) thereof (e.g., IgG1, IgG2, IgG3, IgG4, IgA1 and IgA2), based on the identity of their heavy-chain constant domains referred to as alpha, delta, epsilon, gamma, and mu, respectively.

As used herein, the term "antibody fragment" refers to a portion of an intact antibody and refers to the antigenic determining variable regions of an intact antibody. Examples of antibody fragments include, but are not limited to Fab, Fab', F(ab')2, and Fv fragments, linear antibodies, single chain antibodies, and multispecific antibodies formed from antibody fragments.

"Recombinantly expressed polypeptide" and "recombinant polypeptide" as used herein refer to a polypeptide expressed from a host cell that has been genetically engineered to express that polypeptide. The recombinantly expressed polypeptide can be identical or similar to polypeptides that are normally expressed in the mammalian host cell. The recombinantly expressed polypeptide can also be foreign to the host cell, i.e. heterologous to peptides normally expressed in the mammalian host cell. Alternatively, the recombinantly expressed polypeptide can be chimeric in that portions of the polypeptide contain amino acid sequences that are identical or similar to polypeptides normally expressed in the mammalian host cell, while other portions are foreign to the host cell. As used herein, the terms "recombinantly expressed polypeptide" and "recombinant polypeptide" also encompasses an antibody produced by a hybridoma.

The term "hybridoma" as used herein refers to a cell created by fusion of an immortalized cell derived from an immunologic source and an antibody-producing cell. The resulting hybridoma is an immortalized cell that produces antibodies. The individual cells used to create the hybridoma can be from any mammalian source, including, but not limited to, rat, pig, rabbit, sheep, pig, goat, and human. The term also encompasses trioma cell lines, which result when progeny of heterohybrid myeloma fusions, which are the product of a fusion between human cells and a murine myeloma cell line, are subsequently fused with a plasma cell. Furthermore, the term is meant to include any immortalized hybrid cell line that produces antibodies such as, for example, quadromas (*See, e.g.*, Milstein et al., Nature, 537:3053 (1983)).

### II. CELL CULTURE

The present disclosure provides improved systems for the production of bioproducts, such as proteins and/or polypeptides, by cell culture. The present disclosure provides a method of culturing cells, comprising culturing cells comprising a gene that encodes the bioproduct of interest in a N-1 culture vessel to achieve a high viable cell density (at least 25 x 10⁶ viable cells/ml); inoculating the cells from the N-1 culture vessel into a N culture vessel at a seeding density of at least 8.5 x 10⁶ viable cells/ml; and culturing the cells in the N culture vessel under conditions that allow production of the protein of interest. In some instances , the culture in step (a) is supplemented with nutrients at a level determined based on the viable cell density. In other instances , a cumulative amount of amino acids of about 50 mM to about 2 M is added to the culture of step (a). The present invention describes a method of producing a protein of interest comprising culturing CHO cells, wherein the culture in step (a) is supplemented with nutrients at a level determined based on the viable cell density, as set out in the appended claims.

A typical cell culture process includes inoculation of nutrient media with the starter cells at a seeding density followed by lag phase growth. The lag phase is followed by log phase growth of the culture, ultimately resulting in a plateau phase. The modified cell culture process of the present invention however comprises growing the cells (growth stage) in the N-1 seed-train culture in N-1 culture vessel (N-1 seed-train bioreactor) to obtain high viable cell densities and then taking the cells from the N-1 culture vessel and seeding the cells in the N culture vessel (production bioreactor) at a high viable cell seeding density to produce a bioproduct of interest (production stage).

Various methods of preparing mammalian cells for production of bioproducts, such as proteins or polypeptides, by cell culture (batch, fed-batch, and perfusion culture) are known in the art. A nucleic acid sufficient to achieve expression (typically a vector containing the gene encoding the polypeptide or protein of interest and any operably linked genetic control elements) may be introduced into the host cell line by any number of well-known techniques. Typically, cells are screened to determine which of the host cells have actually taken up the vector and express the polypeptide or protein of interest. Traditional methods of detecting a particular polypeptide or protein of interest expressed by mammalian cells include but are not limited to immunohistochemistry, immunoprecipitation, flow cytometry, immunofluorescence microscopy, SDS-PAGE, Western blots, enzyme-linked immunosorbent assay (ELISA), high performance liquid chromatography (HPLC) techniques, biological activity assays and affinity chromatography. One of ordinary skill in the art will be aware of other appropriate techniques for detecting expressed polypeptides or proteins. If multiple host cells express the polypeptide or protein of interest, some or all of the listed techniques can be used to determine which of the cells expresses that polypeptide or protein at the highest levels.

Once a cell that expresses the polypeptide or protein of interest has been identified, the cell is propagated in culture by any of the variety of methods well-known to one of ordinary skill in the art. The cell expressing the polypeptide or protein of interest is typically propagated by growing it at a temperature and in a medium that is conducive to the survival, growth and viability of the cell.

In the present invention, the cells are propagated/ grown in a N-1 culture vessel to obtain sufficient volume of cells to seed in the production cell culture vessel.

### N-1 Seed-train Cell Culture in N-1 Culture Vessel

Once the N-1 culture vessel has been seeded, the cell culture is maintained in the initial growth phase under conditions conducive to the survival, growth and viability of the cell culture. The precise conditions will vary depending on the cell type, the organism from which the cell was derived, and the nature and character of the expressed polypeptide or protein.

The temperature of the cell culture in the initial growth phase will be selected based primarily on the range of temperatures at which the cell culture remains viable. For example, during the initial growth phase, CHO cells grow well at 37° C. In general, most mammalian cells grow well within a range of about 25° C to 42° C. Preferably, mammalian cells are grown within the range of about 35° C to 40° C. Those of ordinary skill in the art will be able to select appropriate temperature or temperatures in which to grow cells, depending on the needs of the cells and the production requirements of the practitioner.

In one embodiment of the present invention, the temperature of the initial growth phase is maintained at a single, constant temperature. In another embodiment, the temperature of the initial growth phase is maintained within a range of temperatures. For example, the temperature may be steadily increased or decreased during the initial growth phase. Alternatively, the temperature may be increased or decreased by discrete amounts at various times during the initial growth phase. One of ordinary skill in the art will be able to determine whether a single or multiple temperatures should be used, and whether the temperature should be adjusted steadily or by discrete amounts.

The cells may be grown during the initial growth phase for a greater or lesser amount of time, depending on the needs of the practitioner and the requirement of the cells themselves. In one embodiment, the cells are grown for a period of time sufficient to achieve a viable cell density that is a given percentage of the maximal viable cell density that the cells would eventually reach if allowed to grow undisturbed.

The cell culture may be agitated or shaken during the initial culture phase in order to increase oxygenation and dispersion of nutrients to the cells. In accordance with the present invention, one of ordinary skill in the art will understand that it can be beneficial to control or regulate certain internal conditions of the bioreactor during the initial growth phase, including but not limited to pH, temperature, oxygenation, etc. For example, pH can be controlled by supplying an appropriate amount of acid or base and oxygenation can be controlled with sparging devices that are well known in the art.

The culture volume of the N-1 culture vessel can be of any size, but is often smaller than the culture volume of the production cell culture vessel/ bioreactor used in the final growth of cells and production of the polypeptide or protein of interest. The cells may be passaged several times in N-1 seed-train bioreactors of increasing volume prior to seeding the production bioreactor. The cell culture can be agitated or shaken to increase oxygenation of the medium and dispersion of nutrients to the cells. Alternatively or additionally, special sparging devices that are well known in the art can be used to increase and control oxygenation of the culture. In accordance with the present invention, one of ordinary skill in the art will understand that it can be beneficial to control or regulate certain internal conditions of the bioreactor, including but not limited to pH, temperature, oxygenation, etc.

Cell cultures in the initial culture vessel are grown to a desired density before seeding the production culture vessel. It is preferred that most of the cells remain alive prior to seeding, although total or near total viability is not required. In the present invention a cell density of at least 25 x 10⁶ viable cells/ml is achieved. In some embodiments, a cell density of at least 30 x 10⁶ viable cells/ml, at least 35 x 10⁶ viable cells/ml, at least 40 x 10⁶ viable cells/ml, at least 45 x 10⁶ viable cells/ml, at least 50 x 10⁶ viable cells/ml, at least 55 x 10⁶ viable cells/ml, at least 60 x 10⁶ viable cells/ml, at least 65 x 10⁶ viable cells/ml, at least 70 x 10⁶ viable cells/ml, at least 75 x 10⁶ viable cells/ml, at least 80 x 10⁶ viable cells/ml, at least 85 x 10⁶ viable cells/ml, at least 90 x 10⁶ viable cells/ml, at least 95 x 10⁶ viable cells/ml, at least 100 x 10⁶ viable cells/ml, at least 110 x 10⁶ viable cells/ml, at least 110 x 10⁶ viable cells/ml, at least 120 x 10⁶ viable cells/ml, at least 130 x 10⁶ viable cells/ml, at least 135 x 10⁶ viable cells/ml, at least 140 x 10⁶ viable cells/ml, at least 145 x 10⁶ viable cells/ml, or at least 150 x 10⁶ viable cells/ml is achieved.

In one embodiment of the present invention, the cells may be removed from the supernatant, for example, by low-speed centrifugation. It may also be desirable to wash the removed cells with a medium before seeding the next bioreactor to remove any unwanted metabolic waste products or medium components. The medium may be the medium in which the cells were previously grown or it may be a different medium or a washing solution selected by the practitioner of the present invention.

As one of skill in the art can appreciate, different nutrients (of different concentrations of nutrients) may be required in different growth periods of a cell culture cycle. In certain embodiments of the present invention, particular conditions of the growing cell culture may be periodically monitored. As non-limiting example, it may be beneficial or necessary to monitor temperature, pH, cell density, cell viability, integrated viable cell density, lactate levels, ammonium levels, osmolarity, or titer of the expressed polypeptide or protein. Numerous techniques are well known in the art that will allow one of ordinary skill in the art to measure these conditions. For example, cell density may be measured using a hemacytometer, a Coulter counter, or Cell density examination (CEDEX). Viable cell density may be determined by staining a culture sample with Trypan blue. Since only dead cells take up the Trypan blue, viable cell density can be determined by counting the total number of cells, dividing the number of cells that take up the dye by the total number of cells, and taking the reciprocal. HPLC can be used to determine the levels of lactate, ammonium or the expressed polypeptide or protein. Alternatively, the level of the expressed polypeptide or protein can be determined by standard molecular biology techniques such as coomassie staining of SDS-PAGE gels, Western blotting, Bradford assays, Lowry assays, Biuret assays, and UV absorbance. It may also be beneficial or necessary to monitor the post-translational modifications of the expressed polypeptide or protein, including phosphorylation and glycosylation.

One or more of the sample collection device, analytical device, processing program, or feed device can be automated. Thus, e.g., in some embodiments, the methods of the present invention can utilize an automated sample collection device and/or an analytical device that can be off-line, at-line, on-line or in-line, in order to reduce variability, to optimize or enhance quantity and/or quality of a bioproduct during (i.e., in the midst of) any or all steps in a bioproduct manufacturing process.

For purposes of the present description, "off-line" analysis is intended to indicate that a sample is permanently removed from the production process and analyzed apart therefrom at a later point in time such that the data analysis does not convey real-time or near real-time information about in-process conditions. In some embodiments, one or more analytical devices used in the method of the invention is off-line.

For purposes of the present description, "at-line" is intended to indicate that a sample is permanently removed from the production process but is analyzed in a time-frame in close proximity to the time in which it was removed, thereby, providing real-time or near-real time information which may be used to automatically control or change in-process conditions. "At-line" analysis may be performed in an automated or semi-automated fashion. In some embodiments, one or more of the analytical devices used in the method of the present invention is at-line.

For purposes of the present description, "on-line" is intended to indicate that a sample is diverted from the production process, is analyzed in a time-frame in close proximity to the time in which it was removed, thereby, providing real-time or near-real time information which may be used to automatically control or change in-process conditions, and wherein the sample may be (but is not necessarily) returned to the production process. "On-line" analysis may be performed in an automated or semi-automated fashion. In some embodiments, one or more of the analytical devices used in the method of the present invention is on-line.

For purposes of the present description, "in-line" is intended to indicate that a sample is not removed from the production process but, instead, the process "sample" is monitored in real-time by an invasive or non-invasive means, thereby, providing real-time or near-real time information which may be used to automatically control or change in-process conditions. For example, an analytical device (or a sensor-portion connected thereto) may be introduced directly into a bioreactor or purification unit, or the device or sensor-portion may be separated from the bioreactor or purification unit by an appropriate barrier or membrane. "In-line" analysis may be performed in an automated or semi-automated fashion. In-line and on-line analyses are advantageous because they can usually be performed at significantly higher frequency intervals (including continuously or discontinuously) compared to off-line and at-line analytical methods. In some embodiments, one or more of the analytical devices used in the method of the present invention is in-line.

In some embodiments, in order to monitor certain cell culture conditions, it will be necessary to remove small aliquots of the culture for analysis. One of ordinary skill in the art will understand that such removal may potentially introduce contamination into the cell culture, and will take appropriate care to minimize the risk of such contamination.

In an exemplary embodiment of the methods of the present invention, it is envisioned that direct (at-line, in-line, or on-line) determination of one or more marker, e.g., cell viability, in real time from bioreactor samples is done to implement automatic, dynamic feedback controlled addition of supplements, such as amino acid and glucose, and/or removal of waste products, such as ammonium and lactate.

In some embodiments, the viable cell density of the culture in N-1 culture vessel in step (a) is determined about ten times per second. In another embodiment, the viable cell density of the culture in step (a) is determined about nine times per second. In another embodiment, the viable cell density of the culture in step (a) is determined about eight times per second. In another embodiment, the viable cell density of the culture in step (a) is determined about seven times per second. In another embodiment, the viable cell density of the culture in step (a) is determined about six times per second. In another embodiment, the viable cell density of the culture in step (a) is determined about five times per second. In another embodiment, the viable cell density of the culture in step (a) is determined about four times per second. In another embodiment, the viable cell density of the culture in step (a) is determined about three times per second. In another embodiment, the viable cell density of the culture in step (a) is determined about twice per second. In another embodiment, the viable cell density of the culture in step (a) is determined about times per second. In another embodiment, the viable cell density of the culture in step (a) is determined about once per minute. In another embodiment, the viable cell density of the culture in step (a) is determined about once per hour. In another embodiment, the viable cell density of the culture in step (a) is determined about four times per day. In another embodiment, the viable cell density of the culture in step (a) is determined about three times per day. In another embodiment, the viable cell density of the culture in step (a) is determined about twice per day. In another embodiment, the viable cell density of the culture in step (a) is determined about once per day. In other embodiments, the viable cell density of the culture in step (a) is determined continuously. In some embodiments, the viable cell density of the culture is measured via capacitance.

In other embodiments, the viable cell density of the culture in step (a) is determined about once every two seconds, about once every three seconds, about once every four seconds, about once every five seconds, about once every ten seconds, about once every fifteen seconds, about once every twenty seconds, about once every thirty seconds, about once every forty seconds, about once every fifty seconds, about once every minute, about once every two minutes, about once every three minutes, about once every four minutes, about once every five minutes, about once every ten minutes, about once every fifteen minutes, about once every twenty minutes, about once every thirty minutes, about once every hour, about once every two hours, about once every five hours, about four times per day, about twice per day, or about once per day. In other embodiments, the viable cell density of the culture in step (a) is determined continuously.

In some embodiments, waste products, such as ammonium and lactate, are removed during step (a) of the present methods in N-1 culture vessel. In certain aspects, the waste removal is performed by filtration, centrifugation, an inclined cell settler, alternating tangential flow, tangential flow, or combinations thereof.

In the present invention, lactate levels in the cell culture of step (a) are maintained below 15 mM. In other embodiments, lactate levels in the cell culture of step (a) are maintained below 12 mM, 10 mM, 8 mM, 6 mM, 5 mM, 4 mM, 2 mM, 1 mM, 0.5 mM, 0.2 mM, 0.1 mM, or 0.05 mM. In some embodiments, there is no lactate in the cell culture of step (a).

In some instances , the cell culture in the N-1 culture vessel is supplemented with additives (or supplements or nutrients) based on the viable cell density of the culture. An "additive" or "supplement" or "nutrient" can include a single substance, e.g., copper, zinc, amino acid, or can include multiple substances, e.g., zinc and amino acid. The terms "additive" or "supplement" or "supplement" refer to all of the components added, even though they need not be added at the same time, and they need not be added in the same way. For example, one or more components of an "additive" or "supplement" or "nutrient" can be added as a single bolus or two or more boli from a stock solution, while other components of the same "additive" or "supplement" or "nutrient" can be added as part of a feed medium. In addition, any one or more components of an "additive" or "supplement" can be present in the basal medium from the beginning of the cell culture. Nutrient supplementation is determined based on the cell-specific perfusion rate (CSPR). In the present invention, the culture in step (a) is supplemented with nutrients at a level determined based on the viable cell density.

In some embodiments, the nutrients comprise amino acids, zinc, iron, and copper. In some embodiments, zinc is added to the cell culture of step (a) in a cumulative amount of between about 1.8 µM to about 1 mM. In other embodiments, zinc is added to the cell culture of step (a) in a cumulative amount of between about 1.8 µM to about 0.5 mM, about 1.8 µM to about 0.1 mM, about 1.8 µM to about 0.05 mM, about 1.8 µM to about 0.01 mM, about 1.8 µM to about 0.005 mM, about 5 µM to about 1 mM, about 5 µM to about 0.5 mM, about 5 µM to about 0.1 mM, about 5 µM to about 0.05 mM, about 5 µM to about 0.01 mM, about 10 µM to about 1 mM, about 10 µM to about 0.5 mM, about 10 µM to about 0.1 mM, about 10 µM to about 0.05 mM, about 50 µM to about 1 mM, about 50 µM to about 0.5 mM, about 50 µM to about 0.1 mM, about 100 µM to about 1 mM, about 100 µM to about 0.5 mM, about 100 µM to about 0.125 mM, about 200 µM to about 1 mM, about 200 µM to about 0.5 mM, or about 500 µM to about 1 mM.

In some embodiments, iron is added to the cell culture of step (a) in a cumulative amount of between about 0.1 µM to about 20 mM. In other embodiments, iron is added to the cell culture of step (a) in a cumulative amount of between about 0.5 µM to about 20 mM, 0.5 µM to about 10 mM, 0.5 µM to about 5 mM, about 0.5 µM to about 1 mM, about 0.5 µM to about 0.5 mM, about 0.5 µM to about 0.1 mM, about 0.5 µM to about 0.01 mM, about 1 µM to about 20 mM, about 1 µM to about 5 mM, about 1 µM to about 1 mM, about 1 µM to about 0.5 mM, about 1 µM to about 0.1 mM, about 1 µM to about 0.01 mM, about 2 µM to about 20 mM, about 2 µM to about 5 mM, about 2 µM to about 1 mM, about 2 µM to about 0.5 mM, about 2 µM to about 0.1 mM, about 2 µM to about 0.01 mM, 5 µM to about 20 mM, 5 µM to about 10 mM, about 5 µM to about 5 mM, about 5 µM to about 1 mM, about 5 µM to about 0.5 mM, about 5 µM to about 0.1 mM, about 5 µM to about 0.01 mM, 10 µM to about 20 mM, 10 µM to about 10 mM, about 10 µM to about 5 mM, about 10 µM to about 1 mM, about 10 µM to about 0.5 mM, about 10 µM to about 0.1 mM, 50 µM to about 20 mM, 50 µM to about 10 mM, about 50 µM to about 5 mM, about 50 µM to about 1 mM, about 50 µM to about 0.5 mM, about 50 µM to about 0.1 mM, 100 µM to about 20 mM, 100 µM to about 10 mM, about 100 µM to about 5 mM, about 100 µM to about 1 mM, about 100 µM to about 0.5 mM, 1 mM to about 20 mM, 1 mM to about 10 mM, or about 1mM to about 5 mM.

In a preferred embodiment, iron is added to the cell culture of step (a) in a cumulative amount of about 2 µM to about 20 mM in the absence of an iron chelator such as tropolone. In some embodiments, iron is added to the cell culture of step (a) in the absence of an iron chelator in a cumulative amount of about 2 µM to about 20 mM, 2 µM to about 10 mM, 2 µM to about 5 mM, about 2 µM to about 1 mM, about 2 µM to about 0.5 mM, about 2 µM to about 0.1 mM, about 2 µM to about 0.01 mM, 5 µM to about 20 mM, 5 µM to about 10 mM, about 5 uM to about 5 mM, about 5 µM to about 1 mM, about 5 µM to about 0.5 mM, about 5 µM to about 0.1 mM, about 5 µM to about 0.01 mM, 10 µM to about 20 mM, 10 µM to about 10 mM, about 10 µM to about 5 mM, about 10 µM to about 1 mM, about 10 µM to about 0.5 mM, about 10 µM to about 0.1 mM, 50 µM to about 20 mM, 50 µM to about 10 mM, about 50 µM to about 5 mM, about 50 µM to about 1 mM, about 50 µM to about 0.5 mM, about 50 µM to about 0.1 mM, 100 µM to about 20 mM, 100 µM to about 10 mM, about 100 µM to about 5 mM, about 100 µM to about 1 mM, about 100 µM to about 0.5 mM, 1 mM to about 20 mM, 1 mM to about 10 mM, or about 1mM to about 5 mM. In another preferred embodiment, iron is added to the cell culture of step (a) in a cumulative amount of about 0.1 µM to about 20 mM in the presence of an iron chelator. In other embodiments, iron is added to the cell culture of step (a) in the presence of an iron chelator in a cumulative amount of between about 0.1 µM to about 20 mM, 0.1 µM to about 10 mM, 0.1 µM to about 5 mM, about 0.1 µM to about 1 mM, about 0.1 µM to about 0.5 mM, about 0.1 µM to about 0.1 mM, about 0.1 µM to about 0.05 mM, about 0.1 µM to about 0.01 mM, about 0.1 µM to about 0.005 mM, about 0.1 µM to about 0.001 mM, about 1 µM to about 1 mM, about 1 µM to about 5 mM, about 1 µM to about 1 mM, about 1 µM to about 0.5 mM, about 1 µM to about 0.1 mM, about 1 µM to about 0.05 mM, about 1 µM to about 0.01 mM, about 1 µM to about 0.005 mM, about 10 µM to about 1 mM, about 10 µM to about 5 mM, about 10 µM to about 1 mM, about 10 µM to about 0.5 mM, about 10 µM to about 0.1 mM, about 10 µM to about 0.05 mM, about 50 µM to about 1 mM, about 50 µM to about 10 mM, about 50 µM to about 5 mM, about 50 µM to about 1 mM, about 50 µM to about 0.5 mM, about 50 µM to about 0.1 mM, about 100 µM to about 1 mM, about 100 µM to about 5 mM, about 100 µM to about 1 mM, about 100 µM to about 0.5 mM, about 500 µM to about 1 mM, about 500 µM to about 5 mM, about 500 µM to about 2.5 mM, or about 500 µM to about 1 mM.

In some embodiments, copper is added to the cell culture of step (a) in a cumulative amount of between about 0.008 µM to about 1 mM. In other embodiments, copper is added to the cell culture of step (a) in a cumulative about of between about 0.008 µM to about 0.5 mM, about 0.008 µM to about 0.1 mM, about 0.008 µM to about 0.05 mM, about 0.008 µM to about 0.01 mM, about 0.008 µM to about 0.005 mM, 0.008 µM to about 0.5 µM, 0.008 µM to about 0.1 µM, 0.008 µM to about 0.05 µM, 0.008 µM to about 0.01 µM, 0.008 µM to about 0.005 µM, about 0.05 µM to about 1 mM, about 0.05 µM to about 0.5 mM, about 0.05 µM to about 0.1 mM, about 0.05 µM to about 0.05 mM, about 0.05 µM to about 0.01 mM, 0.05 µM to about 0.005 mM, 0.05 µM to about 0.5 µM, about 1 µM to about 1 mM, about 1 µM to about 0.5 mM, about 1 µM to about 0.1 mM, about 1 µM to about 0.01 mM, about 1 µM to about 0.005 mM, 10 µM to about 1 mM, about 10 µM to about 0.5 mM, about 10 µM to about 0.1 mM, about 10 µM to about 0.05 mM, 50 µM to about 1 mM, about 50 µM to about 0.5 mM, about 50 µM to about 0.1 mM, 100 µM to about 1 mM, about 100 µM to about 0.5 mM, about 100 µM to about 0.2 mM, or 500 µM to about 1 mM.

In some embodiments, amino acids are added to the cell culture of step (a) in a cumulative amount of about 50 mM to about 2 M. In another embodiment, amino acids are added to the cell culture of step (a) in a cumulative amount of greater than 50 mM. In certain embodiments, one or more amino acids are added to the cell culture of step (a) in fed-batch or batch culture in a cumulative amount of about 75 mM to about 500 mM. In certain embodiments, one or more amino acids are added to the cell culture of step (a) in fed-batch or batch culture in a cumulative amount of about 50 mM to about 1M, about 50 mM to about 500 mM, or about 50 mM to about 100 mM. In some embodiments, one or more amino acids are added to the cell culture of step (a) in a cumulative amount of about 50 mM, 55 mM, 60 mM, 63 mM, 70 mM, 80 mM, 90 mM, 100 mM, 110 mM, 120 mM, 130 mM, 140 mM, 150 mM, 160 mM, 170 mM, 180 mM, 190 mM, 200 mM, 300 mM, 400 mM, 500 mM, 750 mM, 800 mM, 1M, 1.5 mM, or 2 M. In some embodiments, any cumulative amount of one or more amino acids may be added to the N-1 culture vessel with perfusion culture as there is ammonia build up.

In certain embodiments, the waste products are removed during step (a) and one or more amino acids are added in a cumulative amount of about 50 mM to about 2 M. In other embodiments, the waste products are removed during step (a) and one or more amino acids are added in a cumulative about of about 63 mM to about 200 mM, 63 mM, 70 mM, 80 mM, 90 mM, 100 mM, 110 mM, 120 mM, 130 mM, 140 mM, 150 mM, 160 mM, 170 mM, 180 mM, 190 mM, 200 mM, 300 mM. 400 mM, 500 mM, 750 mM, 800 mM, 1M, 1.5 mM, or 2 M.

In some embodiments, fumarate is added to the cell culture of step (a) in the cumulative amount of about 2 µM to about 20 mM. In other embodiments, fumarate is added to the cell culture of step (a) in a cumulative amount of about 2 µM to about 20 mM, 2 µM to about 10 mM, 2 µM to about 5 mM, about 2 µM to about 1 mM, about 2 µM to about 0.5 mM, about 2 µM to about 0.1 mM, about 2 µM to about 0.01 mM, 5 µM to about 20 mM, 5 µM to about 10 mM, about 5 µM to about 5 mM, about 5 µM to about 1 mM, about 5 µM to about 0.5 mM, about 5 µM to about 0.1 mM, about 5 µM to about 0.01 mM, 10 µM to about 20 mM, 10 µM to about 10 mM, about 10 µM to about 5 mM, about 10 µM to about 1 mM, about 10 µM to about 0.5 mM, about 10 µM to about 0.1 mM, 50 µM to about 20 mM, 50 µM to about 10 mM, about 50 µM to about 5 mM, about 50 µM to about 1 mM, about 50 µM to about 0.5 mM, about 50 µM to about 0.1 mM, 100 µM to about 20 mM, 100 µM to about 10 mM, about 100 µM to about 5 mM, about 100 µM to about 1 mM, about 100 µM to about 0.5 mM, 1 mM to about 20 mM, 1 mM to about 10 mM, or about 1mM to about 5 mM, 0.01 µM, 0.05 µM, 0.1 µM, 0.5 µM, 1.0 µM, 2.0 µM, 5.0 µM, 10 µM, 20 µM, 50 µM, 100 µM, 200 µM, 500 µM, 1 mM, 2 mM, 3 mM, 4 mM, 5 mM, 10 mM, or 20 mM.

In certain embodiments, pH of the culture of step (a) is maintained between about 6.8 to about 7.4. In other embodiments, pH of the culture of step (a) is maintained between about 6.9 to about 7.3. In some embodiments, the desired pH is maintained by titrating the perfusion media pH, without adding a base such as sodium hydroxide or sodium carbonate. In other embodiments, the desired pH is maintained by adding a base such as sodium hydroxide or sodium carbonate.

Cell cultures in the N-1 culture vessel can be cultured in a batch culture, fed-batch culture or a perfusion culture. In one embodiment, the cell culture in the N-1 culture vessel is a batch culture. In another embodiment, the cell culture in the N-1 culture vessel is a fed batch culture.

Typical procedures for producing a polypeptide of interest include batch cultures and fed-batch cultures. A persistent and unsolved problem with traditional batch and fed-batch cultures is the production of metabolic waste products, which have detrimental effects on cell growth, cell viability, and production of expressed polypeptides. Two metabolic waste products that have particularly detrimental effects are lactate and ammonium, which are produced as a result of glucose and glutamine metabolism, respectively. In addition to the enzymatic production of ammonium as a result of glutamine metabolism, ammonium also accumulates in cell cultures as a result of non-metabolic degradation over time. Accordingly, in certain embodiments, the cell culture in the N-1 culture vessel is a perfusion culture. In some embodiments, the perfusion rate for the cell culture in the N-1 culture vessel is between about 0.01 nL/cell/day to about 0.2 nL/cell/day. In other embodiments, the perfusion rate for the cell culture in the N-1 culture vessel is between about 0.01 nL/cell/day to about 0.15 nL/cell/day, about 0.01 nL/cell/day to about 0.1 nL/cell/day, about 0.01 nL/cell/day to about 0.05 nL/cell/day, about 0.05 nL/cell/day to about 0.2 nL/cell/day, about 0.05 nL/cell/day to about 0.15 nL/cell/day, about 0.05 nL/cell/day to about 0.1 nL/cell/day, about 0.1 nL/cell/day to about 0.2 nL/cell/day, about 0.1 nL/cell/day to about 0.15 nL/cell/day, about 0.12 nL/cell/day to about 0.2 nL/cell/day, about 0.12 nL/cell/day to about 0.15 nL/cell/day. In other embodiments, the perfusion rate for the cell culture in the N-1 culture vessel is 0.01 nL/cell/day, 0.02 nL/cell/day, 0.03 nL/cell/day, 0.05 nL/cell/day, 0.07 nL/cell/day, 0.10 nL/cell/day, 0.12 nL/cell/day, 0.14 nL/cell/day, 0.15 nL/cell/day, 0.16 nL/cell/day, 0.18 nL/cell/day, 0.05 nL/cell/day, 0.08 nL/cell/day, 0.1 nL/cell/day, or 0.2 nL/cell/day. In some embodiments, the perfusion in the perfusion culture is alternating tangential flow. In other embodiments, the perfusion in the perfusion cell culture is tangential flow. In some embodiments, the perfusion in the perfusion cell culture uses filtration devices such as hollow fiber and open channel filters.

Typically, the volume of the N-1 culture vessel is less than 4000 liters, less than 3750 liters, less than 3500 liters, less than 3250 liters, less than 3000 liters, less than 2750 liters, less than 2500 liters, less than 2250 liters, less than 2000 liters, less than 1750 liters, less than 1500 liters, less than 1250 liters, or less than 1000 liters. In some embodiments, a N-1 culture vessel is about 50 to about 20,000 liters, about 50 to about 15,000 liters, about 50 to about 10,000 liters, about 50 to about 3570 liters, about 50 to about 3500 liters, about 50 to about 3000 liters, about 50 to about 2500 liters, about 50 to about 2000 liters, about 50 to about 1500 liters, about 50 to about 1000 liters, about 50 to 500 liters, about 100 to about 10,000 liters, about 100 to about 5000 liters, or about 100 to about 4000 liters. In certain embodiments, the N-1 culture vessel is 4000 liters, 3750 liters, 3500 liters, 3250 liters, 3000 liters, 2750 liters, 2500 liters, 2250 liters, 2000 liters, 1750 liters, 1500 liters, 1250 liters, 1000 liters, 750 liters, 500 liters, 250 liters, 200 liters, 100 liters, or 50 liters. The internal conditions of the bioreactor, for example, but not limited to pH and temperature, are typically controlled during the culturing period. The bioreactor can be composed of any material that is suitable for holding mammalian cell cultures suspended in media under the culture conditions of the present invention, including glass, plastic or metal.

Cell cultures in the N-1 culture vessel in step (a) can be maintained for a defined period of time. In some embodiments, the cell culture in step (a) is maintained for about 3 to about 8 days, about 3 to about 7 days, about 3 to about 6 days, about 3 to about 5 days, 4 to about 8 days, about 4 to about 7 days, about 4 to about 6 days, about 4 to about 5 days, about 5 to about 8 days, about 5 to about 7 days, about 5 to about 6 days, about 6 to about 8 days, about 6 to about 7 days, or about 7 to about 8 days. In certain embodiments, the cell culture in step (a) is maintained for 3 days, 4 days, 5 days, 6 days, 7 days, or 8 days.

The cells from step (a) may be diluted to an appropriate density for seeding the production bioreactor in step (b). In a preferred embodiment of the present invention, the cells are diluted into the same medium that will be used in the production bioreactor. Alternatively, the cells can be diluted into another medium or solution, depending on the needs and desires of the practitioner of the present invention or to accommodate particular requirements of the cells themselves, for example, if they are to be stored for a short period of time prior to seeding the production bioreactor.

### Production Cell Culture in N Culture Vessel

The cells grown in the N-1 seed-train culture vessel are taken and seeded/innoculated into a N culture vessel for production cell culture at a high viable cell seeding density. In some embodiments, the N culture vessel is inoculated at a seeding density of 8.5 x 10⁶ viable cells/ml, 10 x 10⁶ viable cells/ml, 15 x 10⁶ viable cells/ml, 20 x 10⁶ viable cells/ml, 25 x 10⁶ viable cells/ml, or 30 x 10⁶ viable cells/ml.

One of ordinary skill in the art will recognize that the production cell culture in the N culture vessel may be a batch, fed-batch or perfusion culture. In certain embodiments, the production culture is a fed-batch culture. In other embodiments, the production culture is a perfusion culture.

Thus, in one embodiment, the cell culture in the N-1 culture vessel and/or the N culture vessel according to a method of the present invention is a batch culture. In another embodiment, the cell culture in the N-1 culture vessel and/or the N culture vessel according to a method of the present invention is a fed batch culture. In a further embodiment, the cell culture the N-1 culture vessel and/or the N culture vessel according to a method of the present invention is a perfusion culture. In some embodiments, the cell culture in the N-1 culture vessel is perfusion culture.

In other embodiments, the culture in the N-1 culture vessel is a fed-batch culture and the culture in the N culture vessel is a batch or a fed-batch culture. In another embodiment, the culture in the N-1 culture vessel is a perfusion culture and the culture in the N culture vessel is a fed-batch or a batch culture. In another embodiment, the culture in the N-1 culture vessel is a batch or a fed-batch culture and the culture in the N culture vessel is a perfusion culture.

Production cell cultures in the N culture vessel in step (b) can be maintained for a defined period of time. In some embodiments, the cell culture in step (b) is maintained for about 8 to about 23 days, about 8 to about 20 days, about 8 to about 15 days, about 10 to about 23 days, about 10 to about 20 days, about 10 to about 15 days, about 15 to about 23 days, about 15 to about 20 days, about 12 to about 23 days, or about 12 to about 20 days. In certain embodiments, the cell culture in step (b) is maintained for 8 days, 10 days, 12 days, 14 days, 16 days, 18 days, 20 days, 22 days, or 23 days.

Typically, the volume of the N culture vessel is between about 200 liters to about 20,000 liters, about 200 liters to about 15,000 liters, about 200 liters to about 12,000 liters, about 200 liters to about 10,000 liters, about 200 liters to about 8,000 liters, about 200 liters to about 5,000 liters, about 1000 liters to about 20,000 liters, about 1000 liters to about 20,000 liters, about 1000 liters to about 15,000 liters, about 1000 liters to about 12,000 liters, about 1000 liters to about 10,000 liters, about 1000 liters to about 8,000 liters, or about 1000 liters to about 5,000 liters. In some embodiments, the volume of the N culture vessel is about 200 liters, about 500 liters, about 800 liters, about 1000 liters, about 1200 liters, about 1500 liters, about 2000 liters, about 2500 liters, about 5000 liters, about 7500 liters, about 10,000 liters, about 12,000 liters, about 15000 liters, about 18000 liters, or about 20,000 liters. In some embodiments, the volume of the N culture vessel is less than or equal to able 20,000 liters.

In one embodiment, a cell culture according to a method of the present invention is a serum-free culture. In another embodiment, a cell culture according to a method of the present invention is a chemically defined culture. In a further embodiment, a cell culture according to a method of the present invention is an animal protein free culture.

### Isolation or Collection of Expressed Polypeptide

The protein of interest produced in the production cell culture is collected from the N culture vessel. In one embodiment, the expressed polypeptide or protein is secreted into the medium and thus cells and other solids can be removed, as by centrifugation or filtering for example, as a first step in the purification process.

Alternatively, the expressed polypeptide can be bound to the surface of the host cell. In this embodiment, the media is removed and the host cells expressing the polypeptide or protein are lysed as a first step in the purification process. Lysis of mammalian host cells can be achieved by any number of means well known to those of ordinary skill in the art, including physical disruption by glass beads and exposure to high pH conditions.

The polypeptide can be isolated and purified by standard methods including, but not limited to, chromatography (e.g., ion exchange, affinity, size exclusion, and hydroxyapatite chromatography), gel filtration, centrifugation, or differential solubility, ethanol precipitation or by any other available technique for the purification of proteins (See, e.g., Scopes, Protein Purification Principles and Practice 2nd Edition, Springer-Verlag, New York, 1987; Higgins, S. J. and Hames, B. D. (eds.), Protein Expression: A Practical Approach, Oxford Univ Press, 1999; and Deutscher, M. P., Simon, M. I., Abelson, J. N. (eds.), Guide to Protein Purification: Methods in Enzymology (Methods in Enzymology Series, Vol 182), Academic Press, 1997). For immunoaffinity chromatography in particular, the protein can be isolated by binding it to an affinity column comprising antibodies that were raised against that protein and were affixed to a stationary support. Alternatively, affinity tags such as an influenza coat sequence, poly-histidine, or glutathione-S-transferase can be attached to the protein by standard recombinant techniques to allow for easy purification by passage over the appropriate affinity column. Protease inhibitors such as phenyl methyl sulfonyl fluoride (PMSF), leupeptin, pepstatin or aprotinin can be added at any or all stages in order to reduce or eliminate degradation of the polypeptide or protein during the purification process. Protease inhibitors are particularly desired when cells must be lysed in order to isolate and purify the expressed polypeptide or protein. One of ordinary skill in the art will appreciate that the exact purification technique will vary depending on the character of the polypeptide or protein to be purified, the character of the cells from which the polypeptide or protein is expressed, and the composition of the medium in which the cells were grown.

### III. CELLS

The present invention describes a method of producing a protein of interest comprising culturing CHO cells, as set out in the appended claims. Non-limiting examples of

mammalian cells that may be used in accordance with the present invention include Chinese hamster ovary cells +/-DHFR (CHO, Urlaub and Chasin, Proc. Natl. Acad. Sci. USA, 77:4216 (1980)). Non-limiting examples of mammalian cells that are not part of the present invention include BALB/c mouse myeloma line (NSO/1, ECACC No: 85110503); human retinoblasts (PER.C6 (CruCell, Leiden, The Netherlands)); monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); baby hamster kidney cells (BHK, ATCC CCL 10);
mouse sertoli cells (TM4, Mather, Biol. Reprod., 23:243-251 (1980)); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TR1 cells (Mather et al., Annals N.Y. Acad. Sci., 383:44-68 (1982)); MRC 5 cells; FS4 cells; and a human hepatoma line (Hep G2).

Disclosed but not part of the invention, the cells are selected from HEK-293 cells, VERO cells, NS0 cells, PER.C6 cells. Sp2/0 cells, BHK cells, MDCK cells, MDBK cells, or COS cells.

Additionally, any number of commercially and non-commercially available hybridoma cell lines that express polypeptides or proteins may be utilized in accordance with the present disclosure. One skilled in the art will appreciate that hybridoma cell lines might have different nutrition requirements and/or might require different culture conditions for optimal growth and polypeptide or protein expression, and will be able to modify conditions as needed.

As noted above, in many instances the cells will be selected or engineered to produce high levels of protein or polypeptide. Often, cells are genetically engineered to produce high levels of protein, for example by introduction of a gene encoding the protein or polypeptide of interest and/or by introduction of control elements that regulate expression of the gene (whether endogenous or introduced) encoding the polypeptide of interest.

Certain polypeptides may have detrimental effects on cell growth, cell viability or some other characteristic of the cells that ultimately limits production of the polypeptide or protein of interest in some way. Even amongst a population of cells of one particular type engineered to express a specific polypeptide, variability within the cellular population exists such that certain individual cells will grow better and/or produce more polypeptide of interest. In certain preferred embodiments of the present invention, the cell line is empirically selected by the practitioner for robust growth under the particular conditions chosen for culturing the cells. In particularly preferred embodiments, individual cells engineered to express a particular polypeptide are chosen for large-scale production based on cell growth, final cell density, percent cell viability, titer of the expressed polypeptide or any combination of these or any other conditions deemed important by the practitioner.

### IV. MEDIA

The cell culture of the present invention is prepared in any medium suitable for the particular cell being cultured. In some embodiments, the medium contains e.g., inorganic salts, carbohydrates (e.g., sugars such as glucose, galactose, maltose or fructose), amino acids, vitamins (e.g., B group vitamins (e.g., B12), vitamin A vitamin E, riboflavin, thiamine and biotin), fatty acids and lipids (e.g., cholesterol and steroids), proteins and peptides (e.g., albumin, transferrin, fibronectin and fetuin), serum (e.g., compositions comprising albumins, growth factors and growth inhibitors, such as, fetal bovine serum, newborn calf serum and horse serum), trace elements (e.g., zinc, copper, selenium and tricarboxylic acid intermediates), hydrolysates (hydrolyzed proteins derived from plant or animal sources), and combinations thereof. Commercially available media such as 5x-concentrated DMEM/F12 (Invitrogen), CD OptiCHO feed (Invitrogen), CD EfficientFeed (Invitrogen), Cell Boost (HyClone), BalanCD CHO Feed (Irvine Scientific), BD Recharge (Becton Dickinson), Cellvento Feed (EMD Millipore), Ex-cell CHOZN Feed (Sigma-Aldrich), CHO Feed Bioreactor Supplement (Sigma-Aldrich), SheffCHO (Kerry), Zap-CHO (Invitria), ActiCHO (PAA/GE Healthcare), Ham's F10 (Sigma), Minimal Essential Medium ([MEM], Sigma), RPMI-1640 (Sigma), and Dulbecco's Modified Eagle's Medium ([DMEM], Sigma) are exemplary nutrient solutions. In addition, any of the media described in Ham and Wallace,(1979) Meth. Enz., 58:44; Barnes and Sato,(1980) Anal. Biochem., 102:255; U.S. Pat. Nos. 4,767,704; 4,657,866; 4,927,762; 5,122,469 or 4,560,655; International Publication Nos. WO 90/03430; and WO 87/00195 can be used as culture media. Any of these media can be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as gentamycin), trace elements (defined as inorganic compounds usually present at final concentrations in the micromolar range) lipids (such as linoleic or other fatty acids) and their suitable carriers, and glucose or an equivalent energy source. In some embodiments the nutrient media is serum-free media, a protein-free media, or a chemically defined media. Any other necessary supplements can also be included at appropriate concentrations that would be known to those skilled in the art.

In one embodiment, a suitable medium contains a basal medium component such as a DMEM/HAM F-12 based formulation (for composition of DMEM and HAM F12 media, see culture media formulations in American Type Culture Collection Catalogue of Cell Lines and Hybridomas, Sixth Edition, 1988, pages 346-349) with modified concentrations of some components such as amino acids, salts, sugar, and vitamins, recombinant human insulin, hydrolyzed peptone, such as Primatone HS or Primatone RL (Sheffield, England), or the equivalent; a cell protective agent, such as Pluronic F68 or the equivalent pluronic polyol; gentamycin; and trace elements.

The present invention provides a variety of media formulations that, when used in accordance with other culturing steps described herein, minimize or prevent decreases in cellular viability in the culture while retaining the ability to manipulate, alter, or change glycosylation of a recombinant glycoprotein of interest.

A media formulation that has been shown to be to useful in manipulating glycosylation, while not having greatly negative impacts on metabolic balance, cell growth and/or viability or on expression of polypeptide or protein comprises the media supplements described herein. One of ordinary skill in the art will understand that the media formulations encompass both defined and non-defined media.

### V. BIOPRODUCTS

### Polypeptides

Any polypeptide that is expressible in a host cell can be produced in accordance with the present invention. The polypeptide can be expressed from a gene that is endogenous to the host cell, or from a gene that is introduced into the host cell through genetic engineering. The polypeptide can be one that occurs in nature, or can alternatively have a sequence that was engineered or selected by the hand of man. An engineered polypeptide can be assembled from other polypeptide segments that individually occur in nature, or can include one or more segments that are not naturally occurring.

Polypeptides that can desirably be expressed in accordance with the present invention will often be selected on the basis of an interesting biological or chemical activity. For example, the present invention can be employed to express any pharmaceutically or commercially relevant enzyme, receptor, antibody, hormone, regulatory factor, antigen, binding agent, etc.

In some embodiments, the protein product of interest is antibody, fusion protein, alpha-synuclein, BART, Lingo, aBDCA2, anti-CD40L, STX-100, Tweak, daclizumab, pegylated interferon, interferon, etanercept, infliximab, trastuzumab, adalimumab, bevacizumab, Tysabri, Avonex, Rituxan, ocrelizumab, obinutuzumab (or anything that binds to CD20).

### Antibodies

Given the large number of antibodies currently in use or under investigation as pharmaceutical or other commercial agents, production of antibodies is of particular interest in accordance with the present invention. Antibodies are proteins that have the ability to specifically bind a particular antigen. Any antibody that can be expressed in a host cell can be used in accordance with the present invention. In one embodiment, the antibody to be expressed is a monoclonal antibody.

Particular antibodies can be made, for example, by preparing and expressing synthetic genes that encode the recited amino acid sequences or by mutating human germline genes to provide a gene that encodes the recited amino acid sequences. Moreover, these antibodies can be produced, e.g., using one or more of the following methods.

Numerous methods are available for obtaining antibodies, particularly human antibodies. One exemplary method includes screening protein expression libraries, e.g., phage or ribosome display libraries. Phage display is described, for example, U.S. Pat. No. 5,223,409; Smith (1985) Science 228:1315-1317; WO 92/18619; WO 91/17271; WO 92/20791; WO 92/15679; WO 93/01288; WO 92/01047; WO 92/09690; and WO 90/02809. The display of Fab's on phage is described, e.g., in U.S. Pat. Nos. 5,658,727; 5,667,988; and 5,885,793.

In addition to the use of display libraries, other methods can be used to obtain an antibody. For example, a protein or a peptide thereof can be used as an antigen in a non-human animal, e.g., a rodent, e.g., a mouse, hamster, or rat.

In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity can be produced and selected. See, e.g., XENOMOUSE^{™}, Green et al. (1994) Nature Genetics 7:13-21, U.S. 2003-0070185, WO 96/34096, and WO 96/33735.

In another embodiment, a monoclonal antibody is obtained from the non-human animal, and then modified, e.g., humanized or deimmunized. Winter describes an exemplary CDR-grafting method that can be used to prepare humanized antibodies described herein (U.S. Pat. No. 5,225,539). All or some of the CDRs of a particular human antibody can be replaced with at least a portion of a non-human antibody. In one embodiment, it is only necessary to replace the CDRs required for binding or binding determinants of such CDRs to arrive at a useful humanized antibody that binds to an antigen.

Humanized antibodies can be generated by replacing sequences of the Fv variable region that are not directly involved in antigen binding with equivalent sequences from human Fv variable regions. General methods for generating humanized antibodies are provided by Morrison, S. L. (1985) Science 229:1202-1207, by Oi et al. (1986) BioTechniques 4:214, and by U.S. Pat. No. 5,585,089; U.S. Pat. No. 5,693,761; U.S. Pat. No. 5,693,762; U.S. Pat. No. 5,859,205; and U.S. Pat. No. 6,407,213. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, can be obtained from a hybridoma producing an antibody against a predetermined target, as described above, from germline immunoglobulin genes, or from synthetic constructs. The recombinant DNA encoding the humanized antibody can then be cloned into an appropriate expression vector. In one embodiment, the expression vector comprises a polynucleotide encoding a glutamine synthetase polypeptide. (See, e.g., Porter et al., Biotechnol Prog 26(5):1446-54 (2010).)

The antibody can include a human Fc region, e.g., a wild-type Fc region or an Fc region that includes one or more alterations. In one embodiment, the constant region is altered, e.g., mutated, to modify the properties of the antibody (e.g., to increase or decrease one or more of: Fc receptor binding, antibody glycosylation, the number of cysteine residues, effector cell function, or complement function). For example, the human IgG1 constant region can be mutated at one or more residues, e.g., one or more of residues 234 and 237. Antibodies can have mutations in the CH2 region of the heavy chain that reduce or alter effector function, e.g., Fc receptor binding and complement activation. For example, antibodies can have mutations such as those described in U.S. Pat. Nos. 5,624,821 and 5,648,260. Antibodies can also have mutations that stabilize the disulfide bond between the two heavy chains of an immunoglobulin, such as mutations in the hinge region of IgG4, as disclosed in the art (e.g., Angal et al. (1993) Mol. Immunol. 30:105-08). See also, e.g., U.S. 2005-0037000.

In other embodiments, the antibody can be modified to have an altered glycosylation pattern (i.e., altered from the original or native glycosylation pattern). As used in this context, "altered" means having one or more carbohydrate moieties deleted, and/or having one or more glycosylation sites added to the original antibody. Addition of glycosylation sites to the presently disclosed antibodies can be accomplished by altering the amino acid sequence to contain glycosylation site consensus sequences; such techniques are well known in the art. Another means of increasing the number of carbohydrate moieties on the antibodies is by chemical or enzymatic coupling of glycosides to the amino acid residues of the antibody. These methods are described in, e.g., WO 87/05330, and Aplin and Wriston (1981) CRC Crit. Rev. Biochem. 22:259-306. Removal of any carbohydrate moieties present on the antibodies can be accomplished chemically or enzymatically as described in the art (Hakimuddin et al. (1987) Arch. Biochem. Biophys. 259:52; Edge et al. (1981) Anal. Biochem. 118:131; and Thotakura et al. (1987) Meth. Enzymol. 138:350). See, e.g., U.S. Pat. No. 5,869,046 for a modification that increases in vivo half-life by providing a salvage receptor binding epitope.

The antibodies can be in the form of full length antibodies, or in the form of fragments of antibodies, e.g., Fab, F(ab')2, Fd, dAb, and scFv fragments. Additional forms include a protein that includes a single variable domain, e.g., a camel or camelized domain. See, e.g., U.S. 2005-0079574 and Davies et al. (1996) Protein Eng. 9(6):531-7.

In one embodiment, the antibody is an antigen-binding fragment of a full length antibody, e.g., a Fab, F(ab')2, Fv or a single chain Fv fragment. Typically, the antibody is a full length antibody. The antibody can be a monoclonal antibody or a mono-specific antibody.

In another embodiment, the antibody can be a human, humanized, CDR-grafted, chimeric, mutated, affinity matured, deimmunized, synthetic or otherwise in vitro-generated antibody, and combinations thereof.

The heavy and light chains of the antibody can be substantially full-length. The protein can include at least one, and preferably two, complete heavy chains, and at least one, and preferably two, complete light chains) or can include an antigen-binding fragment (e.g., a Fab, F(ab')2, Fv or a single chain Fv fragment). In yet other embodiments, the antibody has a heavy chain constant region chosen from, e.g., IgG1, IgG2, IgG3, IgG4, IgM, IgA1, IgA2, IgD, and IgE; particularly, chosen from, e.g., IgG1, IgG2, IgG3, and IgG4, more particularly, IgG1 (e.g., human IgG1). Typically, the heavy chain constant region is human or a modified form of a human constant region. In another embodiment, the antibody has a light chain constant region chosen from, e.g., kappa or lambda, particularly, kappa (e.g., human kappa).

### Receptors

Another class of polypeptides that have been shown to be effective as pharmaceutical and/or commercial agents includes receptors. Receptors are typically trans-membrane glycoproteins that function by recognizing an extra-cellular signaling ligand. Receptors typically have a protein kinase domain in addition to the ligand recognizing domain, which initiates a signaling pathway by phosphorylating target intracellular molecules upon binding the ligand, leading to developmental or metabolic changes within the cell. In one embodiment, the receptors of interest are modified so as to remove the transmembrane and/or intracellular domain(s), in place of which there can optionally be attached an Ig-domain. In one embodiment, receptors to be produced in accordance with the present invention are receptor tyrosine kinases (RTKs). The RTK family includes receptors that are crucial for a variety of functions numerous cell types (see, e.g., Yarden and Ullrich, Ann. Rev. Biochem. 57:433-478, 1988; Ullrich and Schlessinger, Cell 61:243-254, 1990). Non-limiting examples of RTKs include members of the fibroblast growth factor (FGF) receptor family, members of the epidermal growth factor receptor (EGF) family, platelet derived growth factor (PDGF) receptor, tyrosine kinase with immunoglobulin and EGF homology domains-1 (TIE-1) and TIE-2 receptors (Sato et al., Nature 376(6535):70-74 (1995) and c-Met receptor, some of which have been suggested to promote angiogenesis, directly or indirectly (Mustonen and Alitalo, J. Cell Biol. 129:895-898, 1995). Other non-limiting examples of RTK's include fetal liver kinase 1 (FLK-1) (sometimes referred to as kinase insert domain-containing receptor (KDR) (Terman et al., Oncogene 6:1677-83, 1991) or vascular endothelial cell growth factor receptor 2 (VEGFR-2)), fins-like tyrosine kinase-1 (Flt-1) (DeVries et al. Science 255;989-991, 1992; Shibuya et al., Oncogene 5:519-524, 1990), sometimes referred to as vascular endothelial cell growth factor receptor 1 (VEGFR-1), neuropilin-1, endoglin, endosialin, and Ax1. Those of ordinary skill in the art will be aware of other receptors that can be expressed in accordance with the present invention.

### Growth Factors and Other Signaling Molecules

Another class of polypeptides that have been shown to be effective as pharmaceutical and/or commercial agents includes growth factors and other signaling molecules. Growth factors are typically glycoproteins that are secreted by cells and bind to and activate receptors on other cells, initiating a metabolic or developmental change in the receptor cell.

Non-limiting examples of mammalian growth factors and other signaling molecules include cytokines; epidermal growth factor (EGF); platelet-derived growth factor (PDGF); fibroblast growth factors (FGFs) such as aFGF and bFGF; transforming growth factors (TGFs) such as TGF-alpha and TGF-beta, including TGF-beta 1, TGF-beta 2, TGF-beta 3, TGF-beta 4, or TGF-beta 5; insulin-like growth factor-I and -II (IGF-I and IGF-II); des(1-3)-IGF-I (brain IGF-I), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha, -beta, and -gamma; colony stimulating factors (CSFs), e.g., M-CSF, GM-CSF, and G-CSF; interleukins (TLs), e.g., IL-1 to IL-10; tumor necrosis factor (TNF) alpha and beta; insulin A-chain; insulin B-chain; proinsulin; follicle stimulating hormone; calcitonin; luteinizing hormone; glucagon; anti-clotting factors such as Protein C; atrial natriuretic factor; lung surfactant; a plasminogen activator, such as urokinase or human urine or tissue-type plasminogen activator (t-PA); bombesin; thrombin, hemopoietic growth factor; enkephalinase; RANTES (regulated on activation normally T-cell expressed and secreted); human macrophage inflammatory protein (MIP-1-alpha); mullerian-inhibiting substance; relaxin A-chain; relaxin B-chain; prorelaxin; mouse gonadotropin-associated peptide; neurotrophic factors such as bone-derived neurotrophic factor (BDNF), neurotrophin-3, -4, -5, or -6 (NT-3, NT-4, NT-5, or NT-6), or a nerve growth factor such as NGF-beta. One of ordinary skill in the art will be aware of other growth factors or signaling molecules that can be expressed in accordance with the present invention.

### Clotting Factors

In some embodiments, the protein of interest comprises a clotting factor. Clotting factor, as used herein, means any molecule, or analog thereof, which prevents or decreases the duration of a bleeding episode in a subject with a hemostatic disorder. For example, a clotting factor for the invention can be a full-length clotting factor, a mature clotting factor, or a chimeric clotting factor. In other words, it means any molecule having clotting activity. Clotting activity, as used herein, means the ability to participate in a cascade of biochemical reactions that culminates in the formation of a fibrin clot and/or reduces the severity, duration or frequency of hemorrhage or bleeding episode. Examples of clotting factors can be found in U.S. Pat. No. 7,404,956.

In one embodiment, the clotting factor is Factor VIII, Factor IX, Factor XI, Factor XII, fibrinogen, prothrombin, Factor V, Factor VII, Factor X, Factor XIII or von Willebrand Factor. The clotting factor can be a factor that participates in the extrinsic pathway. The clotting factor can be a factor that participates in the intrinsic pathway. Alternatively, the clotting factor can be a factor that participates in both the extrinsic and intrinsic pathway.

In one embodiment, the clotting factor can be a human clotting factor or a non-human clotting factor, e.g., derived from a non-human primate, a pig or any mammal. The clotting factor can be chimeric clotting factor, e.g., the clotting factor can comprise a portion of a human clotting factor and a portion of a porcine clotting factor or a portion of a first non-human clotting factor and a portion of a N non-human clotting factor.

In another embodiment, the clotting factor can be an activated clotting factor. Alternatively, the clotting factor can be an inactive form of a clotting factor, e.g., a zymogen. The inactive clotting factor can undergo activation subsequent to being linked to at least a portion of an immunoglobulin constant region. The inactive clotting factor can be activated subsequent to administration to a subject. Alternatively, the inactive clotting factor can be activated prior to administration.

In certain embodiments, the clotting factor is a Factor VIII protein. "Factor VIII protein" or "FVIII protein" as used herein, means functional Factor VIII protein in its normal role in coagulation, unless otherwise specified. Thus, the term FVIII includes variant proteins that are functional. In one embodiment, the FVIII protein is the human, porcine, canine, rat, or murine FVIII protein. A functional FVIII protein can be a fusion protein, such as, but not limited to, a fusion protein comprising a fully or partially B-domain deleted FVIII, at least a portion of an immunoglobulin constant region, e.g., an Fc domain, or both. Myriad functional FVIII variants have been constructed and can be used as recombinant FVIII proteins as described herein. See PCT Publication Nos. WO 2011/069164 A2, WO 2012/006623 A2, WO 2012/006635 A2, or WO 2012/006633 A2.

A great many functional FVIII variants are known. In addition, hundreds of nonfunctional mutations in FVIII have been identified in hemophilia patients. See, e.g., Cutler et al., Hum. Mutat. 19:274-8 (2002). In addition, comparisons between FVIII from humans and other species have identified conserved residues that are likely to be required for function. See, e.g., Cameron et al., Thromb. Haemost. 79:317-22 (1998) and US 6,251,632.

In certain aspects, a recombinant FVIII protein of the invention is chimeric. A "chimeric protein," or "chimeric polypeptide" as used herein, means a protein or polypeptide that includes within it at least two stretches of amino acids from different sources, e.g., a FVIII protein comprising a heterologous moiety. In one embodiment, the heterologous moiety can be a half-life extending moiety. Examples of the heterologous moieties include, but are not limited to, an immunoglobulin constant region or a fragment thereof, e.g., an Fc region or an FcRn binding partner, a VWF molecule, or a fragment thereof, albumin, albumin binding polypeptide, Fc, PAS, the β subunit of the C-terminal peptide (CTP) of human chorionic gonadotropin, polyethylene glycol (PEG), hydroxyethyl starch (HES), albumin-binding small molecules, or combinations thereof. In some embodiments, the chimeric protein is a FVIII monomer dimer hybrid.

A long-acting or long-lasting FIX polypeptide useful for the invention is a chimeric polypeptide comprising a FIX polypeptide and an FcRn binding partner. The FIX polypeptide of the invention comprises a functional Factor IX polypeptide in its normal role in coagulation, unless otherwise specified. Thus, the FIX polypeptide includes variant polypeptides that are functional and the polynucleotides that encode such functional variant polypeptides. In one embodiment, the FIX polypeptides are the human, bovine, porcine, canine, feline, and murine FIX polypeptides. The full length polypeptide and polynucleotide sequences of FIX are known, as are many functional variants, e.g., fragments, mutants and modified versions. FIX polypeptides include full-length FIX, full-length FIX minus Met at the N-terminus, full-length FIX minus the signal sequence, mature FIX (minus the signal sequence and propeptide), and mature FIX with an additional Met at the N-terminus. FIX can be made by recombinant means ("recombinant Factor IX" or "rFIX"), i.e., it is not naturally occurring or derived from plasma.

The clotting factor can also include a FIX protein or any variant, analog, or functional fragments thereof. A great many functional FIX variants are known. International publication number WO 02/040544 A3, discloses mutants that exhibit increased resistance to inhibition by heparin at page 4, lines 9-30 and page 15, lines 6-31. International publication number WO 03/020764 A2, discloses FIX mutants with reduced T cell immunogenicity in Tables 2 and 3 (on pages 14-24), and at page 12, lines 1-27. International publication number WO 2007/149406 A2, discloses functional mutant FIX molecules that exhibit increased protein stability, increased in vivo and in vitro half-life, and increased resistance to proteases at page 4, line 1 to page 19, line 11. WO 2007/149406 A2 also discloses chimeric and other variant FIX molecules at page 19, line 12 to page 20, line 9. International publication number WO 08/118507 A2, discloses FIX mutants that exhibit increased clotting activity at page 5, line 14 to page 6, line 5. International publication number WO 09/051717 A2, discloses FIX mutants having an increased number of N-linked and/or O-linked glycosylation sites, which results in an increased half-life and/or recovery at page 9, line 11 to page 20, line 2. International publication number WO 09/137254 A2, also discloses Factor IX mutants with increased numbers of glycosylation sites at page 2, paragraph [006] to page 5, paragraph [011] and page 16, paragraph [044] to page 24, paragraph [057]. International publication number WO 09/130198 A2, discloses functional mutant FIX molecules that have an increased number of glycosylation sites, which result in an increased half-life, at page 4, line 26 to page 12, line 6. International publication number WO 09/140015 A2, discloses functional FIX mutants that an increased number of Cys residues, which may be used for polymer (e.g., PEG) conjugation, at page 11, paragraph [0043] to page 13, paragraph [0053]. The FIX polypeptides are also described in International Application No. PCT/US2011/043569 filed July 11, 2011 and published as WO 2012/006624 on January 12, 2012.

In addition, hundreds of non-functional mutations in FIX have been identified in hemophilia patients, many of which are disclosed in Table 1, at pages 11-14 of International publication number WO 09/137254 A2. Such non-functional mutations are not included in the invention, but provide additional guidance for which mutations are more or less likely to result in a functional FIX polypeptide.

In some embodiments, the chimeric protein of the invention is a FIX monomer dimer hybrid. Monomer-dimer hybrid can comprise two polypeptide chains, one chain comprising a FIX polypeptide and a first Fc region, and another chain comprising, consisting essentially of, or consisting of a second Fc region. In certain aspects, a FIX monomer dimer hybrid consists essentially of or consists of two polypeptide chains, a first chain consisting essentially of or consisting of a FIX polypeptide and a second chain consisting essentially of or consisting of a second Fc region.

In some embodiments, a clotting factor is a mature form of Factor VII or a variant thereof. Factor VII (FVII, F7; also referred to as Factor 7, coagulation factor VII, serum factor VII, serum prothrombin conversion accelerator, SPCA, proconvertin and eptacog alpha) is a serine protease that is part of the coagulation cascade. FVII includes a Gla domain, two EGF domains (EGF-1 and EGF-2), and a serine protease domain (or peptidase S1 domain) that is highly conserved among all members of the peptidase S1 family of serine proteases, such as for example with chymotrypsin. FVII occurs as a single chain zymogen, an activated zymogen-like two-chain polypeptide and a fully activated two-chain form.

Exemplary FVII variants include those with increased specific activity, e.g., mutations that increase the activity of FVII by increasing its enzymatic activity (Kcat or Km). Such variants have been described in the art and include, e.g., mutant forms of the molecule as described for example in Persson et al. 2001. PNAS 98:13583; Petrovan and Ruf. 2001. J. Biol. Chem. 276:6616; Persson et al. 2001 J. Biol. Chem. 276:29195; Soejima et al. 2001. J. Biol. Chem. 276:17229; Soejima et al. 2002. J. Biol. Chem. 247:49027.

In one embodiment, a variant form of FVII includes the mutations. Exemplary mutations include V158D-E296V-M298Q. In another embodiment, a variant form of FVII includes a replacement of amino acids 608-619 (LQQSRKVGDSPN, corresponding to the 170- loop) from the FVII mature sequence with amino acids EASYPGK from the 170-loop of trypsin. High specific activity variants of FIX are also known in the art. For example, Simioni et al. (2009 N.E. Journal of Medicine 361:1671) describe an R338L mutation. Chang et al. (1988 JBC 273:12089) and Pierri et al. (2009 Human Gene Therapy 20:479) describe an R338A mutation. Other mutations are known in the art and include those described, e.g., in Zogg and Brandstetter. 2009 Structure 17:1669; Sichler etl al. 2003. J. Biol. Chem. 278:4121; and Sturzebecher et al. 1997. FEBS Lett 412:295.

Full activation, which occurs upon conformational change from a zymogen-like form, occurs upon binding to is co-factor tissue factor. Also, mutations can be introduced that result in the conformation change in the absence of tissue factor. Hence, reference to FVIIa includes both two-chain forms thereof: the zymogen-like form (e.g., activatable FVII), and the fully activated two-chain form.

Various patents or applications disclose examples of the clotting factors useful for the invention . For example, various monomer dimer hybrid constructs comprising clotting factors (FVII, FIX, and FVIII) are disclosed in US 7,404,945, US 7,348,004, US 7,862,820, US 8,329,182, and US 7,820,162. Examples of FVIII chimeric protein are additionally disclosed in US Appl. Nos. 61/734,954 or 61/670,553. Examples of FVII chimeric protein are disclosed in US Appl. No. 61/657,688.

### G-Protein Coupled Receptors

Another class of polypeptides that have been shown to be effective as pharmaceutical and/or commercial agents includes growth factors and other signaling molecules. G-protein coupled receptors (GPCRs) are proteins that have seven transmembrane domains. Upon binding of a ligand to a GPCR, a signal is transduced within the cell which results in a change in a biological or physiological property of the cell.

GPCRs, along with G-proteins and effectors (intracellular enzymes and channels which are modulated by G-proteins), are the components of a modular signaling system that connects the state of intracellular second messengers to extracellular inputs. These genes and gene-products are potential causative agents of disease.

The GPCR protein superfamily now contains over 250 types of paralogues, receptors that represent variants generated by gene duplications (or other processes), as opposed to orthologues, the same receptor from different species. The superfamily can be broken down into five families: Family I, receptors typified by rhodopsin and the beta2-adrenergic receptor and currently represented by over 200 unique members; Family II, the recently characterized parathyroid hormone/calcitonin/secretin receptor family; Family III, the metabotropic glutamate receptor family in mammals; Family IV, the cAMP receptor family, important in the chemotaxis and development of D. discoideum; and Family V, the fungal mating pheromone receptors such as STE2.

### Viruses

The present invention describes a method of producing a protein of interest, as set out in the appended claims. The present disclosure also provides methods for the production of viruses using a cell culture according to methods known to those of skill in the field of virology. The viruses to be produced in accordance with the present disclosure can be chosen from the range of viruses known to infect the cultured cell type. For instance, when utilizing a mammalian cell culture, viruses can be chosen from the genera of orthomyxoviruses, paramyxoviruses, reoviruses, picornaviruses, flaviviruses, arenaviruses, herpesviruses, poxviruses, coronaviruses and adenoviruses. The virus used can be a wild-type virus, an attenuated virus, a reassortant virus, or a recombinant virus. In addition, instead of actual virions being used to infect the cells with a virus, an infectious nucleic acid clone can be utilized according to infectious clone transfection methods known to those of skill in the field of virology. In one instance, the virus produced is an influenza virus.

The foregoing description is to be understood as being representative only and is not intended to be limiting. Alternative methods and materials for implementing the invention and also additional applications will be apparent to one of skill in the art.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis et al. U.S. Pat. No: 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Mayer and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Maryland (1989).

General principles of antibody engineering are set forth in Antibody Engineering, 2nd edition, C.A.K. Borrebaeck, Ed., Oxford Univ. Press (1995). General principles of protein engineering are set forth in Protein Engineering, A Practical Approach, Rickwood, D., et al., Eds., IRL Press at Oxford Univ. Press, Oxford, Eng. (1995). General principles of antibodies and antibody-hapten binding are set forth in: Nisonoff, A., Molecular Immunology, 2nd ed., Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (eds), Basic and Clinical -Immunology (8th ed.), Appleton & Lange, Norwalk, CT (1994) and Mishell and Shiigi (eds), Selected Methods in Cellular Immunology, W.H. Freeman and Co., New York (1980).

Standard reference works setting forth general principles of immunology include Current Protocols in Immunology, John Wiley & Sons, New York; Klein, J., Immunology: The Science of Self-Nonself Discrimination, John Wiley & Sons, New York (1982); Kennett, R., et al., eds., Monoclonal Antibodies, Hybridoma: A New Dimension in Biological Analyses, Plenum Press, New York (1980); Campbell, A., "Monoclonal Antibody Technology" in Burden, R., et al., eds., Laboratory Techniques in Biochemistry and Molecular Biology, Vol. 13, Elsevere, Amsterdam (1984), Kuby Immunology 4th ed. Ed. Richard A. Goldsby, Thomas J. Kindt and Barbara A. Osborne, H. Freemand & Co. (2000); Roitt, I., Brostoff, J. and Male D., Immunology 6th ed. London: Mosby (2001); Abbas A., Abul, A. and Lichtman, A., Cellular and Molecular Immunology Ed. 5, Elsevier Health Sciences Division (2005); Kontermann and Dubel, Antibody Engineering, Springer Verlan (2001); Sambrook and Russell, Molecular Cloning: A Laboratory Manual. Cold Spring Harbor Press (2001); Lewin, Genes VIII, Prentice Hall (2003); Harlow and Lane, Antibodies: A Laboratory Manual, Cold Spring Harbor Press (1988); Dieffenbach and Dveksler, PCR Primer Cold Spring Harbor Press (2003).

### Examples

The example below use three different CHO cell lines that produce three different recombinant proteins to show that the use of high density perfusion N-1 technology coupled with high-seed fed-batch production can increase protein harvest titer and maximize manufacturing capacity compared to the traditional cell culture process with low density batch N-1 followed by a low-seed fed-batch production.

The high-seed production cultures allowed the same or higher harvest titer in a shorter amount of time, thus improving manufacturing output and capacity. Furthermore, the gains in output resulted in desirable product quality attributes for all three of our proteins of interest. The process intensification offered by the perfusion N-1/high-seed fed-batch production combination can either yield product quality that is comparable to that of the low-seed fed-batch production or it can result in product quality that is different from and superior to that of the low-seed.

### Example 1

### Cell Lines and Culture Methods

### Cell Lines and Media

Three different CHO cell lines producing three different recombinant protein products were used in these experiments. Cell lines A and B produce monoclonal antibodies and cell line C produces an Fc fusion protein. The chemically-defined basal and feed media used in this study were as described in Huang et al., "Maximizing productivity of CHO cell-based fed-batch culture using chemically defined media conditions and typical manufacturing equipment." Biotechnology Progress 2010, 26, (5), 1400-10.

### Seed Cultures

All three cell lines were thawed and grown as previously described (Huang *et al.*, supra, and Kshirsagar et al., "Controlling trisulfide modification in recombinant monoclonal antibody produced in fed-batch cell culture." Biotechnology and Bioengineering 2012, 109, (10), 2523-32.) Cells were passaged in 1L or 3L shake flasks (Corning, NY) every 3-4 days using 1x-concentrated basal media with incubator settings of 36°C and 5% CO₂.

Cells from the shake flasks were then used to inoculate bench-scale 5-L glass Applikon^{®} bioreactors (Foster City, CA) equipped with Finesse DeltaV^{™} controllers (San Jose, CA) in either the traditional batch N-1 mode or the perfusion N-1 mode for the culture stage preceding the production bioreactor. For both seed train bioreactor modes, temperature was controlled at 36°C, pH was controlled between 6.9 and 7.3 using 1M sodium carbonate (batch mode) or 0.5M sodium hydroxide (perfusion mode) and CO₂ sparging, and agitation varied between 200 rpm to 400 rpm.

For "traditional" batch N-1 (the control culture), 1x-concentrated basal media was used. There was no feed, only base addition for pH control. Dissolved oxygen was controlled at 30% using air and oxygen sparging. The N-1 bioreactor had a 2.5-L working volume and the culture duration was three days. At the end of the N-1 stage, cells were split into traditional low-seed fed-batch production bioreactors at a target seeding density of 0.4 x 10⁶ vc/mL or 1 x 10⁶ vc/mL.

For perfusion N-1, 1x- and 2x-concentrated basal media were used, depending on the cell line and application. We used alternating tangential flow (ATF4^{™}, Refine Technology, Pine Brook, NJ) perfusion technology with 0.2 µm filters to retain the cells but not the protein product. The perfusion media rates varied between a cell-specific perfusion rate (CSPR) of 0.05 and 0.12 nL cell⁻¹ day⁻¹, depending on the cell line and the media. The ATF exchange rate varied between 1.0 to 2.5 L/min. The volumetric perfusion flow rate, equal to the ATF filter harvest rate, was automatically controlled by a biocapacitance probe (Aber, Aberystwyth, UK; described in Dowd et al., "Optimization and control of perfusion cultures using a viable cell probe and cell specific perfusion rates." Cytotechnology 2003, 42, (1), 35-45).

Weight-based feedback control via a Sartorius Signum^{®} scale (Sartorius, Gottingen, Germany) was used to deliver fresh perfusion media to the reactor to maintain a total working volume of 4L throughout the duration of the perfusion N-1 stage. Dissolved oxygen was controlled at 50% using air and oxygen sparging to compensate for the residence time inside the ATF unit. The perfusion N-1 culture duration was five or seven days. At the end of the perfusion N-1 stage, cells were split into high-seed fed-batch production bioreactors.

### Fed-Batch Production Culture Methods

The fed-batch production bioreactors were also performed in 5-L Applikon bioreactors with a 2.5-L initial working volume. All fed-batch production bioreactors for all cell lines used the same 1x-concentrated basal and feed media. Feed media additions commenced on Day 2 or Day 3 for the low-seed process and Day 0 of the high-seed process. Feed was administered every 24 hours; the feed volume was proportional to the integral of viable cells (IVC) of the corresponding process.

For cell lines A and B, temperature was controlled at 35°C, pH was controlled between 6.9 and 7.3 using 1M sodium carbonate and CO2 sparging, and agitation varied between 300 rpm to 400 rpm. The low-seed culture duration was 17-days, while the high-seed culture duration was 12-days. The high-seed production bioreactors for cell line A were seeded at 10 x 10⁶ vc/mL; 25x the seed density of the corresponding low-seed production bioreactors.

For cell line C, the low-seed process consisted of a 6-day period where the temperature was controlled at 35°C followed by an 8-day period where the temperature was controlled at 30°C. The high-seed cell line C process was seeded at 10 x 10⁶ vc/mL; 10x the seed density of the corresponding low-seed process. The high-seed process was carried out for a 12 days and temperature was controlled at 30°C starting from Day 0. The pH for both high and low-seed was controlled between 6.9 and 7.3 using 1M sodium carbonate and CO₂ sparging, and agitation varied between 200 and 300 rpm.

### Offline Analytical Methods

Daily bioreactor sampling was performed to measure metabolic parameters including glucose, glutamine, glutamate, lactate, ammonium, sodium, potassium, and calcium using the NOVA Bioprofile^{®} Flex biosensor (NOVA Biomedical, Waltham, MA). Viable cell density (VCD) and viability were measured using Trypan blue exclusion via an automatic Cedex Cell Counter (Innovatis AG, Germany). pH and pCO2 were measured using a NOVA pHOX Analyzer (NOVA Biomedical, Waltham, MA). Supernatant samples were sterile filtered and stored at 2-8°C for further titer and product quality analyses. IVC was determined from the area under the viable cell density curve and is estimated by using a sum of trapezoids approximation across the desired time interval.

### Analysis of Protein Concentration and Product Quality

Protein concentrations for cell lines A, B, and C were measured using an HPLC system (Waters, MA) with a UV detector and a Protein G affinity column (Applied Biosystems, CA). Small scale purification was performed on the harvest cell culture fluid using Protein A affinity chromatography and the corresponding product quality assays were performed on the Protein A eluate. For the antibodies produced by cell lines A and B, the percentage of the main monomer peak in the Protein A eluate was measured using the LabChip GXII (Perkin Elmer, Waltham, MA) under non-reducing impurity profiling conditions. Imaging capillary isoelectric focusing (ICIEF) was used to measure the antibody charge heterogeneity. Hydrophilic interaction ultra performance liquid chromatography (HILIC-UPLC) was used to measure the antibody N-glycan profile.

For the Fc fusion protein produced by cell line C, HPLC hydrophobic interaction chromatography (HIC) was performed on the Protein A eluate to quantify the active and inactive levels of the recombinant protein.

### Example 2

### Cell Line A: N-1 Perfusion Optimization

Cell line A was used as the model system for perfusion N-1 optimization. In the first iteration of perfusion N-1, we performed a 7-day N-1 perfusion culture using a 1x-concentrated chemically-defined basal media. The cell-specific perfusion rate (CSPR) was 0.1 nL cell⁻¹ day⁻¹. Cells grew exponentially to greater than 60 x 10⁶ vc/mL and maintained high viability (FIG. 1A). However, the initial perfusion N-1 process was sub-optimal due to high accumulation of pCO₂ and high consumption of the perfusion media (FIG. 1C, D).

We first sought to reduce the volume of perfusion media perfused by reducing the CSPR. Reducing the CSPR greatly reduced the media consumption, but led to stunted growth and did not resolve the pCO₂ accumulation. Furthermore, the 0.05 nL cell⁻¹ day⁻¹ CSPR had to be increased to 0.07 during the culture due to sub-optimal (poor) growth.

After observing sub-optimal results at 0.05 nL cell⁻¹ day⁻¹ CSPR using 1x-concentrated media, we used a 2x-concentrated version of our basal media. The more concentrated media combined with a higher N-1 seed density (2 x 10⁵ vc/mL instead of the original seed density of 1 x 10⁵ vc/mL) allowed us to shorten the culture duration from 7 days to 5 days and enabled the use of a lower 0.05 nL cell⁻¹ day⁻¹ CSPR for exponential growth to greater than 40x10⁶ vc/mL. In order to seed the N-1 higher, we progressively increased the starting seed densities of each of the preceding inoculum passages. Furthermore, the shorter culture duration kept pCO₂ lower than 100mmHg and the 2x concentrated media resulted in the consumption of less than 3 bioreactor volumes of perfusion media (FIG. 1). On the fifth and final day of the perfusion N-1 stage, we split the cells into high-seed fed-batch production bioreactors.

### Example 3

### Cell Line A: High-Seed Fed-Batch Production

High-seed (10 x 10⁶ vc/mL) fed-batch production cultures were fed daily using chemically-defined feed media as described above. If we align Day 0 of the high-seed process with Day 5 of the low-seed process, the performance of the high-seed cultures track that of the low-seed culture in VCD, viability, glucose consumption, lactate generation, and protein production (FIG. 2). The high-seed process was able to reach the same harvest titer as the low-seed process, generating 5 g/L of antibody in just 12 days, compared with 17 days for the low-seed process. Furthermore, protein aggregation and charge heterogeneity were not affected by high-seed culture conditions *(see* Table 1, below).

### Example 4

### Cell Line B: High-Seed Fed-Batch Production

Cell line A's optimized perfusion N-1 process using 2x-concentrated media, higher N-1 seed density, and shorter culture duration was applied to cell line B with similar success. Growth in the perfusion N-1 stage was exponential and resembled that of the optimized process for cell line A, also generating growth to approximately 40 x 10⁶ vc/mL in 5 days. At the end of the 5-day N-1 stage, cells were split into high-seed (10 x 10⁶ vc/mL) fed-batch production bioreactor cultures. For cell line B, the higher seeding density resulted in much higher growth (FIG. 3A). Since the cells were fed based on growth, the overall glucose consumption and lactate generation trended well with that of the low-seed cultures, which were also fed based on growth (FIG. 3B, C). Like cell line A, the high-seed process for cell line B generated the same amount of protein in five fewer days (FIG. 3D). Furthermore, these efficiency gains did not come at the expense of product quality, as aggregation, charge heterogeneity, and glycosylation of the high-seed harvest material were similar to that of the low-seed harvest material (Table 1).

**Table 1: Normalized product quality attributes for cell line A and B.**

| **Cell Line** | **N-1 Format** | **N Seed Density** | **GXII Major Peak** | **Charge Heterogeneity** | | | **N-glycan Profile** | | |
|---|---|---|---|---|---|---|---|---|---|
| | | | | **%Acidic** | **%Main** | **%Basic** | **%G0F** | **%G1F** | **%G2F** |
| A | Batch | Low | 100.0±0.2 | 100±2 | 100±1 | 100±22 | 100 | 100 | 100 |
| A | Perfusion | High | 97.3±0.5 | 90±2 | 110±1 | 118±47 | 100±1 | 110±7 | 122±10 |
| B | Batch | Low | 100 | 100±7 | 100±20 | 100±6 | 100 | 100 | 100 |
| B | Perfusion | High | 99.7±0.1 | 119±2 | 130±23 | 81±2 | 100±2 | 102±2 | 101±4 |

In Table 1, 100% represents the average for the low-seed production processes for cell lines A and B and the values for the high-seed production processes were portrayed relative to 100%. All data were N=2, except the low seed N-glycan data. data. All attributes were obtained using the Protein A eluate. Purity measured by LabChip GXII protein profiling, charge heterogeneity measured by ICIEF, and N-linked glycosylation measured by HILIC-UPLC do not differ greatly between low-seed and high-seed harvest material for cell lines A and B.

### Example 5

### Impact on Increasing Manufacturing Capacity

By lengthening the N-1 seed train stage and seeding the production bioreactor higher, we can shorten the production stage and create more balance between seed train and production bioreactor occupancies. This provides similar or greater product output by weight in less time. This would debottleneck the production bioreactor and allow for more runs to be performed in the time allotted for a campaign, and thus, higher levels of protein production given the same constraints.

The extent of the debottlenecking obtained by implementing a perfusion N-1 process depends on the duration of the seed and production stages as well as the facility layout. For instance, if one seed train (S) vessel feeds one production (P) vessel (P/S ratio = 1), the production vessel would remain the bottleneck in a 5-day perfusion N-1/12-day fed-batch production scenario (FIG. 4A). If one seed train vessel feeds two production vessels (P/S ratio = 2), the bottleneck is then shifted to the N-1 stage (FIG. 4B). As the P/S ratio increases to 3, the bottleneck increasingly shifts toward the seed train and the benefit of switching to perfusion N-1 seed culture diminishes (FIG. 4C). By plotting the output as a function of the number of production vessels per seed train, we see that the benefit obtained by having multiple production vessels per seed train plateaus regardless of whether the seed cultures are batch or perfusion (FIG. 4D). Even the traditional, short batch seed train becomes the bottleneck at P/S ratios above 3. To maximize the number of batches, the optimal P/S ratio for a 3-day N-1/17-day N process (low-seed) is 3 while the optimal ratio for a 5-day N-1/12-day N (high-seed) is 2.

Based on the facility layout models in FIG. 4, we can calculate the increase in output as a result of switching to the perfusion N-1/high-seed fed-batch for cell lines A and B. Since Biogen Idec manufacturing facilities are either P/S = 1 or P/S = 2, we can implement perfusion N-1 to lengthen the N-1 and shorten the production stage to obtain more batches in the same time. Based on those assumptions and the high-seed fed-batch data for cell lines A and B, we can potentially increase manufacturing output by greater than 30% depending on the facility of choice (Table 2). A greater relative change in capacity is obtained using P/S = 1 facilities while a greater absolute mass of protein produced is obtained using P/S = 2 facilities.

**Table 2: Theoretical gains in fed-batch production manufacturing output obtained by implementing perfusion N-1 cultures for cell lines A and B.**

| **N-1 Format** | **N Seed Density** | **Harvest Titer** | **VPR g L⁻¹ day⁻¹** | **P/S Ratio** | **Production BR Harvest Volume (L)** | **Batches per Year** | **kg mAb per Year** | **Percent Increase** |
|---|---|---|---|---|---|---|---|---|
| Batch | Low | 5 g/L | 0.29 | 1 | 2,000-L | 18 | 180 kg | |
| Perfusion | High | 5 g/L | 0.42 | 1 | 2,000-L | 24 | 240 kg | +33% |
| Batch | Low | 5 g/L | 0.29 | 2 | 15,000-L | 35 | 2,625 kg | |
| Perfusion | High | 5 g/L | 0.42 | 2 | 15,000-L | 44 | 3,300 kg | +26% |

Assumptions made for the calculations in Table 2: One seed train per production bioreactor, campaign length is one-year, maximum number of batches per one-year campaign slot, 2-day turnaround time per bioreactor.

### Example 6

### Cell Line C: N-1 Perfusion Optimization

Perfusion N-1 was also performed with cell line C. The optimized perfusion N-1 process could not be used effectively with cell line C due to poor growth and viability (FIG. 5). Thus, we reverted to the original formulation of 1x-concentrated basal media as the perfusion media with a higher CSPR of 0.10-0.12 nL cell⁻¹ day⁻¹. Using these parameters, the cell line C perfusion N-1 stage reached greater than 35x10⁶ vc/mL in 6 days with high viability (FIG. 5). On Day 6, the perfusion N-1 culture was split into high-seed fed-batch production bioreactors at 10 x 10⁶ vc/mL.

### Example 7

### Cell Line C: High-Seed Fed-Batch Production and Impact on Product Quality

The Fc fusion protein-of-interest produced by cell line C is prone to misfolding, which leads to accumulation of the protein in an inactive form. Since lower temperatures are more thermodynamically favorable for protein folding (discussed in Onuchic, J. N.; Luthey-Schulten, Z.; Wolynes, P. G., Theory of protein folding: the energy landscape perspective. Annual review of physical chemistry 1997, 48, 545-600), a common protein expression technique for alleviating misfolding is to reduce the culture temperature (discussed in Rodriguez et al., "Enhanced production of monomeric interferon-beta by CHO cells through the control of culture conditions." Biotechnology Progress 2005, 21, (1), 22-30; and Gasser et al., "Protein folding and conformational stress in microbial cells producing recombinant proteins: a host comparative overview." Microbial Cell Factories 2008, 7, 11.) Thus, the low-seed cell line C process employs a change in temperature from 35°C to 30°C during the culture. Since the VCD of the low-seed process on the day of the temperature change is approximately the same VCD as that of the seed density of the high-seed process, the high-seed process was directly inoculated at a 30°C culture temperature on Day 0. This resulted in different growth, metabolism, and protein production profiles (FIG. 6).

By directly culturing the cell line C high-seed process at 30°C, we produced a greater proportion of the active species compared to that of the low-seed process (*see* Table 3, below). The use of the 0.5 µm hollow fiber filter enabled the inactive protein produced during the higher temperature 36°C perfusion N-1 stage to perfuse out of the N-1 vessel. Thus, there was very little carryover of the inactive species when the perfusion N-1 was split into high-seed fed-batch production. The high-seed culture could then commence fed-batch production at a more thermodynamically favorable temperature of 30°C starting on Day 0 without sacrificing cell mass. As expected, the lower production temperature reduced the amount of inactive species, which in turn boosted the level of the active species.

**Table 3: Product quality attributes and active titer for cell line C.**

| **N Seed Density** | **%Active Species** | **%Inactive Species** | **Total Titer (g/L)** | **Active Titer (g/L)** | **%Active Titer Increase** |
|---|---|---|---|---|---|
| Low | 54.1 | 45.9 | 2.1 | 1.1 | |
| High | 64.3 | 35.7 | 2.0 | 1.3 | +18% |

For Table 3, HIC-HPLC was used to determine the relative percentage of active and inactive protein. All data were N=1. The two species sum to 100%. Active titer is defined as the proportion of the total titer that is comprised of the active species. Directly seeding cell line C at 30°C greatly enhances the production of active species, which correspondingly increased the amount of active titer. The lower temperature also reduced the level of inactive species.

Having generally described this invention, a further understanding can be obtained by reference to the examples provided herein. These examples are for purposes of illustration only and are not intended to be limiting.

All of the various aspects, embodiments, and options described herein can be combined in any and all variations.

## Claims

1. A method of producing a protein of interest, comprising:
(a) culturing CHO cells comprising a gene that encodes the protein of interest in a N-1 culture vessel to achieve a cell density of at least 25 x 10⁶ viable cells/ml;
(b) inoculating a N culture vessel at a seeding density of at least 8.5 x 10⁶ viable cells/ml with cells obtained from step (a); and
(c) culturing the cells in the N culture vessel under conditions that allow production of the protein of interest,
wherein the culture in step (a) is supplemented with nutrients at a level determined based on the viable cell density,
wherein waste products are removed during step (a) and/or the culture in the N-1 culture vessel of step (a) is perfused, and
wherein lactate levels are maintained below 15 mM in the culture of step (a).

2. The method of claim 1, wherein, during step (a), the viable cell density of the culture is periodically determined.

3. The method of claim 1 or claim 2, wherein the nutrients comprise amino acids, zinc, iron, and copper.

4. The method of any one of claims 1-3, wherein the waste removal is performed by filtration, centrifugation, inclined cell settler, alternating tangential flow, or tangential flow.

5. The method of any one of claims 1-4, wherein a cell density of at least 30 x 10⁶ viable cells/ml, at least 35 x 10⁶ viable cells/ml, at least 40 x 10⁶ viable cells/ml, at least 50 x 10⁶ viable cells/ml, or at least 60 x 10⁶ viable cells/ml is achieved during step (a).

6. The method of any one of claims 2-5, wherein the viable cell density of the culture in step (a) is determined 1-10 times per second.

7. The method of any one of claims 1-6, wherein the culture in N-1 culture vessel of step (a) is perfused and the perfusion rate is 0.01 nL/cell/ day to 0.2 nL/cell/day.

8. The method of any one of claims 1-7, further comprising the step of collecting the protein of interest produced by the cells in the N culture vessel.

9. The method of any one of claims 1-8, wherein:
(i) the culture of step (c) is performed in fed-batch mode; or
(ii) the culture in N culture vessel of step (c) is perfused.

10. The method of any one of claims 1-9, wherein the protein of interest is an antibody, fusion protein, alpha-synuclein, BART, Lingo, aBDCA2, anti-CD40L, STX-100, Tweak, daclizumab, pegylated interferon, interferon, etanercept, infliximab, trastuzumab, adalimumab, bevacizumab, Tysabri, Avonex, Rituxan, ocrelizumab, obinutuzumab or anything that binds to CD20.

11. The method of any one of claims 1-10, wherein the N culture vessel is inoculated at a seeding density of at least 8.5 x 10⁶ viable cells/ml, at least 10 x 10⁶ viable cells/ml, 15 x 10⁶ viable cells/ml, 20 x 10⁶ viable cells/ml, 25 x 10⁶ viable cells/ml or 30 x 10⁶ viable cells/ml.

12. The method of any one of claims 1-11, wherein:
(i) the culture of step (a) is maintained for 3 days to 8 days; and/or
(ii) the culture of step (c) is maintained for 8 days to 23 days.

13. The method of any one of claims 1-12, wherein (a) the volume of the N-1 culture vessel is between 50 liters to 20,000 liters, between 50 liters to 10,000 liters, between 100 liters to 10,000 liters, or between 100 liters to 4,000 liters; and/or (b) the volume of the N culture vessel is between 200 liters to 20,000 liters, between 200 liters to 10,000 liters, between 1000 liters to 10,000 liters, or between 1000 liters to 5,000 liters.

14. The method of any one of claims 1-13, wherein lactate levels are maintained below 10 mM, below 1 mM or below 0.1 mM in the culture of step (a).

15. The method of any one of claims 1-14, wherein pH of the culture of step (a) is maintained between 6.8 to 7.4 or between 6.9 to 7.3.

## Patentansprüche

1. Verfahren zum Herstellen eines Proteins von Interesse, umfassend:
(a) Kultivieren von CHO-Zellen, umfassend ein Gen, das das Protein von Interesse in einem N-1 Kulturgefäß kodiert, um eine Zelldichte von mindestens 25 x 10⁶ lebensfähigen Zellen/ml zu erreichen;
(b) Impfen eines N Kulturgefäßes mit einer Aussaatdichte von mindestens 8,5 x 10⁶ lebensfähigen Zellen/ml mit den in Schritt (a) erlangten Zellen; und
(c) Kultivieren der Zellen in dem N Kulturgefäß unter Bedingungen, die eine Produktion des Proteins von Interesse ermöglichen,
wobei die Kultur in Schritt (a) mit Nährstoffen in einem Umfang ergänzt wird, der basierend auf der Dichte der lebensfähigen Zellen bestimmt wird,
wobei Abfallprodukte während des Schritts (a) entfernt werden und/oder die Kultur in dem N-1 Kulturgefäß des Schritts (a) perfundiert wird und
wobei Laktatwerte in der Kultur von Schritt (a) unter 15 mM aufrechterhalten werden.

2. Verfahren nach Anspruch 1, wobei während des Schritts (a) die Dichte der lebensfähigen Zellen in der Kultur periodisch bestimmt wird.

3. Verfahren nach Anspruch 1 oder 2, wobei die Nährstoffe Aminosäuren, Zink, Eisen und Kupfer umfassen.

4. Verfahren nach einem der Ansprüche 1-3, wobei die Abfallbeseitigung durch Filtration, Zentrifugation, Schrägzellenabsetzer, alternierende tangentiale Strömung oder tangentiale Strömung durchgeführt wird.

5. Verfahren nach einem der Ansprüche 1-4, wobei während des Schritts (a) eine Zelldichte von mindestens 30 x 10⁶ lebensfähigen Zellen/ml, mindestens 35 x 10⁶ lebensfähigen Zellen/ml, mindestens 40 x 10⁶ lebensfähigen Zellen/ml, mindestens 50 x 10⁶ lebensfähigen Zellen/ml oder mindestens 60 x 10⁶ lebensfähigen Zellen/ml erreicht wird.

6. Verfahren nach einem der Ansprüche 2-5, wobei die Dichte der lebensfähigen Zellen der Kultur in Schritt (a) 1-10 Mal pro Sekunde bestimmt wird.

7. Verfahren nach einem der Ansprüche 1-6, wobei die Kultur in dem N-1 Kulturgefäß von Schritt (a) perfundiert wird und die Perfusionsrate 0,01 nl/Zelle/Tag bis 0,2 nl/Zelle/Tag ist.

8. Verfahren nach einem der Ansprüche 1-7, ferner umfassend den Schritt des Sammelns des von den Zellen in dem N Kulturgefäß produzierten Proteins von Interesse.

9. Verfahren nach einem der Ansprüche 1-8, wobei:
(i) die Kultur von Schritt (c) in einem Fed-Batch-Modus durchgeführt wird; oder
(ii) die Kultur in dem N Kulturgefäß von Schritt (c) perfundiert wird.

10. Verfahren nach einem der Ansprüche 1-9, wobei das Protein von Interesse ein Antikörper, Fusionsprotein, alpha-Synuclein, BART, Lingo, aBDCA2, anti-CD40L, STX-100, Tweak, Daclizumab, pegyliertes Interferon, Interferon, Etanercept, Infliximab, Trastuzumab, Adalimumab, Bevacizumab, Tysabri, Avonex, Rituxan, Ocrelizumab, Obinutuzumab oder etwas, das an CD20 bindet, ist.

11. Verfahren nach einem der Ansprüche 1-10, wobei das N Kulturgefäß mit einer Aussaatdichte von mindestens 8,5 x 10⁶ lebensfähigen Zellen/ml, mindestens 10 x 10⁶ lebensfähigen Zellen/ml, 15 x 10⁶ lebensfähigen Zellen/ml, 20 x 10⁶ lebensfähigen Zellen/ml, 25 x 10⁶ lebensfähigen Zellen/ml oder 30 x 10⁶ lebensfähigen Zellen/ml geimpft wird.

12. Verfahren nach einem der Ansprüche 1-11, wobei:
(i) die Kultur von Schritt (a) 3 Tage bis 8 Tage lang aufrechterhalten wird; und/oder
(ii) die Kultur von Schritt (c) 8 Tage bis 23 Tage lang aufrechterhalten wird.

13. Verfahren nach einem der Ansprüche 1-12, wobei (a) das Volumen des N-1 Kulturgefäßes zwischen 50 Litern bis 20.000 Litern, zwischen 50 Litern bis 10.000 Litern, zwischen 100 Litern bis 10.000 Litern oder zwischen 100 Litern bis 4.000 Litern ist; und/oder (b) das Volumen des N Kulturgefäßes zwischen 200 Litern bis 20.000 Litern, zwischen 200 Litern bis 10.000 Litern, zwischen 1.000 Litern bis 10.000 Litern oder zwischen 1.000 Litern bis 5.000 Litern ist.

14. Verfahren nach einem der Ansprüche 1-13, wobei Laktatwerte in der Kultur von Schritt (a) unter 10 mM, unter 1 mM oder unter 0,1 mM aufrechterhalten werden.

15. Verfahren nach einem der Ansprüche 1-14, wobei ein pH-Wert der Kultur von Schritt (a) zwischen 6,8 und 7,4 oder zwischen 6,9 und 7,3 aufrechterhalten wird.

## Revendications

1. Procédé de production d'une protéine d'intérêt, comprenant :
(a) la culture de cellules CHO comprenant un gène codant pour la protéine d'intérêt dans un récipient de culture N-1 pour atteindre une densité cellulaire d'au moins 25 x 10⁶ cellules viables/ml ;
(b) l'inoculation d'un récipient de culture N à une densité d'ensemencement d'au moins 8,5 x 10⁶ cellules viables/ml avec des cellules obtenues à l'étape (a) ; et
(c) la culture des cellules dans le récipient de culture N dans des conditions permettant la production de la protéine d'intérêt,
dans lequel la culture de l'étape (a) est complétée par des nutriments à un niveau déterminé sur la base de la densité de cellules viables,
dans lequel les produits résiduaires sont éliminés pendant l'étape (a) et/ou la culture dans le récipient de culture N-1 de l'étape (a) est perfusée, et
dans lequel les niveaux de lactate sont maintenus en dessous de 15 mM dans la culture de l'étape (a).

2. Procédé selon la revendication 1, dans lequel, pendant l'étape (a), la densité de cellules viables de la culture est déterminée périodiquement.

3. Procédé selon la revendication 1 ou la revendication 2, dans lequel les nutriments comprennent des acides aminés, du zinc, du fer et du cuivre.

4. Procédé selon l'une quelconque des revendications 1 à 3, dans lequel l'élimination des produits résiduaires est réalisée par filtration, centrifugation, décanteur cellulaire incliné, flux tangentiel alterné ou flux tangentiel.

5. Procédé selon l'une quelconque des revendications 1 à 4, dans lequel une densité cellulaire d'au moins 30 x 10⁶ cellules viables/ml, d'au moins 35 x 10⁶ cellules viables/ml, d'au moins 40 x 10⁶ cellules viables/ml, d'au moins 50 x 10⁶ cellules viables/ml ou d'au moins 60 x 10⁶ cellules viables/ml est atteinte pendant l'étape (a).

6. Procédé selon l'une quelconque des revendications 2 à 5, dans lequel la densité de cellules viables de la culture à l'étape (a) est déterminée 1 à 10 fois par seconde.

7. Procédé selon l'une quelconque des revendications 1 à 6, dans lequel la culture dans un récipient de culture N-1 de l'étape (a) est perfusée et le débit de perfusion est de 0,01 nL/cellule/jour à 0,2 nL/cellule/jour.

8. Procédé selon l'une quelconque des revendications 1 à 7, comprenant également l'étape de collecte de la protéine d'intérêt produite par les cellules dans le récipient de culture N.

9. Procédé selon l'une quelconque des revendications 1 à 8, dans lequel :
(i) la culture de l'étape (c) est réalisée en mode à écoulement discontinu ; ou
(ii) la culture dans le récipient de culture N de l'étape (c) est perfusée.

10. Procédé selon l'une quelconque des revendications 1 à 9, dans lequel la protéine d'intérêt est un anticorps, une protéine de fusion, l'alpha-synucléine, BART, Lingo, aBDCA2, un anti-CD40L, STX-100, Tweak, le daclizumab, un interféron pégylé, un interféron, l'étanercept, l'infliximab, le trastuzumab, l'adalimumab, le bévacizumab, le Tysabri, l'Avonex, le Rituxan, l'ocrélizumab, l'obinutuzumab ou toute substance se liant au CD20.

11. Procédé selon l'une quelconque des revendications 1 à 10, dans lequel le récipient de culture N est inoculé à une densité d'ensemencement d'au moins 8,5 x 10⁶ cellules viables/ml, d'au moins 10 x 10⁶ cellules viables/ml, de 15 x 10⁶ cellules viables/ml, de 20 x 10⁶ cellules viables/ml, de 25 x 10⁶ cellules viables/ml ou de 30 x 10⁶ cellules viables/ml.

12. Procédé selon l'une quelconque des revendications 1 à 11, dans lequel :
(i) la culture de l'étape (a) est maintenue pendant 3 à 8 jours ; et/ou
(ii) la culture de l'étape (c) est maintenue pendant 8 à 23 jours.

13. Procédé selon l'une quelconque des revendications 1 à 12, dans lequel (a) le volume du récipient de culture N-1 est compris entre 50 litres et 20 000 litres, entre 50 litres et 10 000 litres, entre 100 litres et 10 000 litres, ou entre 100 litres et 4 000 litres ; et/ou (b) le volume du récipient de culture N est compris entre 200 litres et 20 000 litres, entre 200 litres et 10 000 litres, entre 1 000 litres et 10 000 litres, ou entre 1 000 litres et 5 000 litres.

14. Procédé selon l'une quelconque des revendications 1 à 13, dans lequel les niveaux de lactate sont maintenus en dessous de 10 mM, en dessous de 1 mM ou en dessous de 0,1 mM dans la culture de l'étape (a).

15. Procédé selon l'une quelconque des revendications 1 à 14, dans lequel le pH de la culture de l'étape (a) est maintenu entre 6,8 et 7,4 ou entre 6,9 et 7,3.
